# EUROPEAN PATENT SPECIFICATION

(11) **EP 2 720 696 B1**
(45) Date of publication and mention of the grant of the patent: **25.05.2016**
(21) Application number: 12730099.4
(22) Date of filing: 13.06.2012
(51) Int. Cl.: A61K 31/4045, A61K 31/519

(54) **COMBINATION OF PANOBINOSTAT AND RUXOLITINIB IN THE TREATMENT OF CANCER SUCH AS A MYELOPROLIFERATIVE NEOPLASM**
KOMBINATION AUS PANOBINOSTAT UND RUXOLITINIB BEI DER BEHANDLUNG VON KREBS WIE ETWA EINEM MYELOPROLIFERATIVEN NEOPLASMA
COMBINAISON DE PANOBINOSTAT ET DE RUXOLITINIB DANS LE TRAITEMENT DU CANCER TEL QU'UN NÉOPLASME MYÉLOPROLIFÉRATIF

(30) Priority: 14.06.2011 US 201161496750 P; 09.12.2011 US 201161568717 P
(43) Date of publication of application: 23.04.2014
(73) Proprietor: Novartis AG, 4056 Basel (CH)
(72) Inventor: BAFFERT, Fabienne, CH-4002 Basel (CH); RADIMERSKI, Thomas, CH-4002 Basel (CH); GADBAW, Brian, East Hanover, New Jersey 07936-1080 (US)
(74) Representative: Kristl, Jernej
(86) International application number: PCT/US2012/042174
(87) International publication number: WO 2012/174061

(56) References cited:
- SMITH JASON ET AL: "Upregulated JAK/STAT Signaling Represents a Major Mode of Resistance to HDAC Inhibition In Lymphoma and Provides a Rationale for Novel Combination Therapy (Abstract 434)", American Society of Hematology 52nd ASH Annual Meeting and Exposition (December 4-7, 2010), 6 December 2010 (2010-12-06), XP002681787, ORLANDO, FL, USA Retrieved from the Internet: URL:https://ash.confex.com/ash/2010/webpro gram/Paper34411.html [retrieved on 2012-08-14]
- A. QUINTAS-CARDAMA ET AL: "Preclinical characterization of the selective JAK1/2 inhibitor INCB018424: therapeutic implications for the treatment of myeloproliferative neoplasms", BLOOD, vol. 115, no. 15, 15 April 2010 (2010-04-15), pages 3109-3117, XP055035457, ISSN: 0006-4971, DOI: 10.1182/blood-2009-04-214957
- Y. WANG ET AL: "Cotreatment with panobinostat and JAK2 inhibitor TG101209 attenuates JAK2V617F levels and signaling and exerts synergistic cytotoxic effects against human myeloproliferative neoplastic cells", BLOOD, vol. 114, no. 24, 3 December 2009 (2009-12-03), pages 5024-5033, XP055035448, ISSN: 0006-4971, DOI: 10.1182/blood-2009-05-222133
- WANG YONGCHAO ET AL: "Co-Treatment with JAK2 Inhibitor TC101209 and Panobinostat or hsp90 Inhibitor AUY922 Attenuates Mutant JAK2-V617F Levels and Activity in Human Myeloproliferative Disorder Cells", BLOOD, vol. 112, no. 11, November 2008 (2008-11), pages 1281-1282, XP009161993, & 50TH ANNUAL MEETING OF THE AMERICAN- SOCIETY-OF-HEMATOLOGY; SAN FRANCISCO, CA, USA; DECEMBER 06 -09, 2008 ISSN: 0006-4971
- SRIDURGA MITHRAPRABHU ET AL: "Deactylase inhibition in myeloproliferative neoplasms", INVESTIGATIONAL NEW DRUGS ; THE JOURNAL OF NEW ANTICANCER AGENTS, vol. 28, no. 1, 3 December 2010 (2010-12-03), pages 50-57, XP019869088, KLUWER ACADEMIC PUBLISHERS, BO ISSN: 1573-0646, DOI: 10.1007/S10637-010-9590-4
- Baffert Fabienne et al: "Improved Efficacy Upon Combined JAK1/2 and Pan-Deacetylase Inhibition Using Ruxolitinib (INC424) and Panobinostat (LBH589) in Preclinical Mouse Models of JAK2V617F-Driven Disease", American Society of Hematology 53rd ASH Annual Meeting and Exposition (December 10-13, 2011), 12 December 2011 (2011-12-12), XP002681788, SAN DIEGO, CA, USA Retrieved from the Internet: URL:https://ash.confex.com/ash/2011/webpro gram/Paper37458.html [retrieved on 2012-08-14]

## Description

### Field of Invention

The invention relates to a combination which comprises:
(a) Compound A (name: (R)-3-(4-(7H-pyrrolo[2,3-d]pyrimidin-4-yl)-1H-pyrazol-1-yl)-3-cyclopentylpropanenitrile) of Formula (A): 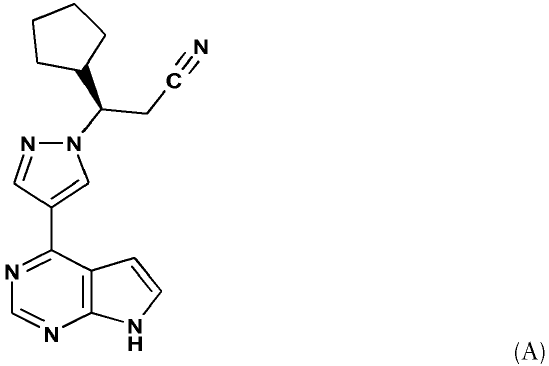 or a pharmaceutically acceptable salt thereof; and
(b) Compound B (name: *N*-hydroxy-3-[4-[[[2-(2-methyl-1*H*-indol-3-yl)-ethyl]-amino]methyl]phenyl]-2*E*-2-propenamide) of Formula (B): 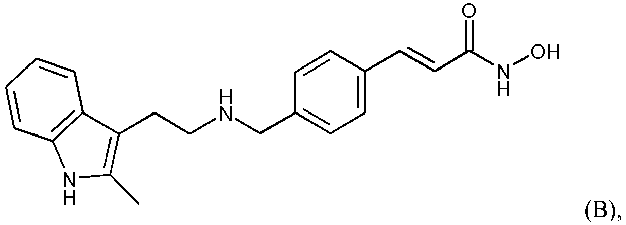 or a pharmaceutically acceptable salt thereof;
for simultaneous, concurrent, separate or sequential use, especially for use in the treatment of proliferative diseases. The invention also relates to pharmaceutical compositions comprising such a combination and to a method of treating proliferative diseases, in a mammal, particularly a human, with such a combination. The present invention further also relates to a commercial package or product comprising such a combination.

### Background

Wang et al. reported in a paper (Blood, 2009) that panobinostat (PS) treatment depleted the autophosphorylation, expression, and downstream signaling of JAK2V617F. Treatment with PS also disrupted the chaperone association of JAK2V617F with hsp90, promoting proteasomal degradation of JAK2V617F. PS also induced apoptosis of the cultured JAK2V617F-expressing human erythroleukemia HEL92.1.7 and Ba/F3-JAK2V617F cells. Treatment with the JAK2 tyrosine kinase inhibitor TG101209 attenuated JAK2V617F autophosphorylation and induced apoptosis of HEL92.1.7 and Ba/F3-JAK2V617F cells. Cotreatment with PS and TG101209 further depleted JAK/STAT signaling and synergistically induced apoptosis of HEL92.1.7 and Ba/F3-JAK2V617F cells. Cotreatment with TG101209 and PS exerted greater cytotoxicity against primary CD34+ myeloproliferative neoplasm cells than normal CD34+ hematopoietic progenitor cells.

Myeloproliferative neoplasms (MPNs) are a group of disorders that cause an overproduction of blood cells (platelets, white blood cells and red blood cells) in the bone marrow. MPNs include polycythemia vera (PV), primary or essential thrombocythemia (ET), primary or idiopathic myelofibrosis, chronic myelogenous (myelocytic) leukemia (CML), chronic neutrophilic leukemia (CNL), juvenile myelomonocytic leukemia (JML) and chronic eosinophilic leukemia (CEL)/hyper eosinophilic syndrome (HES). These disorders are grouped together because they share some or all of the following features: involvement of a multipotent hematopoietic progenitor cell, dominance of the transformer clone over the non-transformed hematopoietic progenitor cells, overproduction of one or more hematopoietic lineages in the absence of a definable stimulus, growth factor-independent colony formation in vitro, marrow hypercellularity, megakaryocyte hyperplasia and dysplasia, abnormalities predominantly involving chromosomes 1, 8, 9, 13, and 20, thrombotic and hemorrhagic diatheses, exuberant extramedullary hematopoiesis, and spontaneous transformation to acute leukemia or development of marrow fibrosis but at a low rate, as compared to the rate in CML. The incidence of MPNs varies widely, ranging from approximately 3 per 100,000 individuals older than 60 years annually for CML to 0.13 per 100,000 children from birth to 14 years annually for JML (Vardiman JW et al., Blood 100 (7): 2292-302, 2002).

Accordingly, there remains a need for new treatments of MPNs, as well as other cancers.

### Summary of the Invention

Provided herein is a combination therapy comprising a JAK inhibitor Compound A of Formula (A) or a pharmaceutically acceptable salt thereof; and histone deacetylase (HDAC) inhibitor Compound B of Formula (B) or a pharmaceutically acceptable salt thereof. The combination therapy is useful for the treatment of a variety of cancers. The combination therapy is also useful for the treatment of any number of JAK-associated diseases and/or HDAC associated diseases. Compound A of Formula (A) is also known as ruxolitinib. Compound B of Formula (B) is also known as panobinostat.

The combination therapy provided herein is useful for the treatment of a JAK-associated disease in a subject. Accordingly, in one aspect, provided herein is a composition for use in the treatment of cancer in a subject in need thereof comprising administering to the subject an effective amount of the composition discussed above. In one embodiment, the cancer is a myeloproliferative neoplasm. Non-limiting examples of myeloproliferative neoplasms that can be treated using the combination therapy of the invention include, but are not limited to, chronic myeloid leukemia (CML), polycythemia vera (PV), essential thrombocythemia (ET), primary or idiopathic myelofibrosis (PMF), chronic neutrophilic leukemia, chronic eosinophilic leukemia, chronic myelomonocytic leukemia, juvenile myelomonocytic leukemia, hypereosinophilic syndrome, systemic mastocytosis, and atypical chronic myelogenous leukemia.

In one embodiment of these compositions for use in the treatment, the subject is human.

In another embodiment, the treatment comprises co-administering Compound A and Compound B.

In another embodiment, Compound A and Compound B are in a single formulation or unit dosage form.

In still another embodiment, the treatment comprises administering Compound A and Compound B at substantially the same time, or different times.

In another embodiment of the method, Compound A is administered to the subject, followed by administration of Compound B.

In still another embodiment, Compound B is administered to the subject, followed by administration of the Compound A.

In another embodiment of the composition for use in the treatment, Compound A and/or Compound B is administered at amounts that would not be effective when one or both is administered alone, but which amounts are effective in combination.

### Brief Description of Drawing

Figure 1 shows the body weight over the Compound A and/or Compound B treatment period.
Figures 2 and 3 show the effect of treatment of BioL levels after 7 treatment days, i.e., day 11 after cell injection (treatment started on day 4 post cell injection).
Figure 4 shows the spleen weight after 8 days of treatment.
Figures 5 and 6 show the result of PD-marker analysis (spleen extracts) 2 hours post therapy.

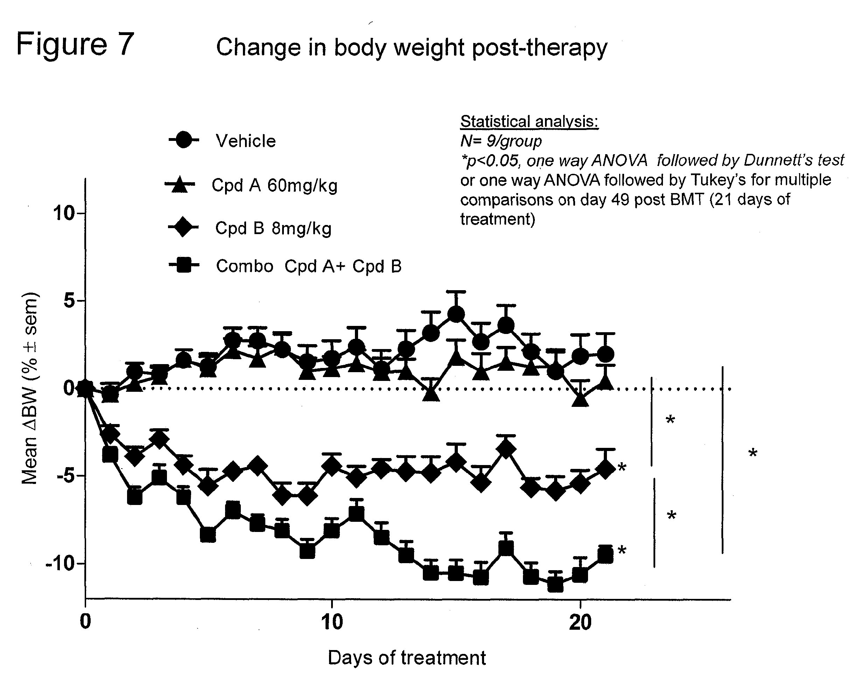

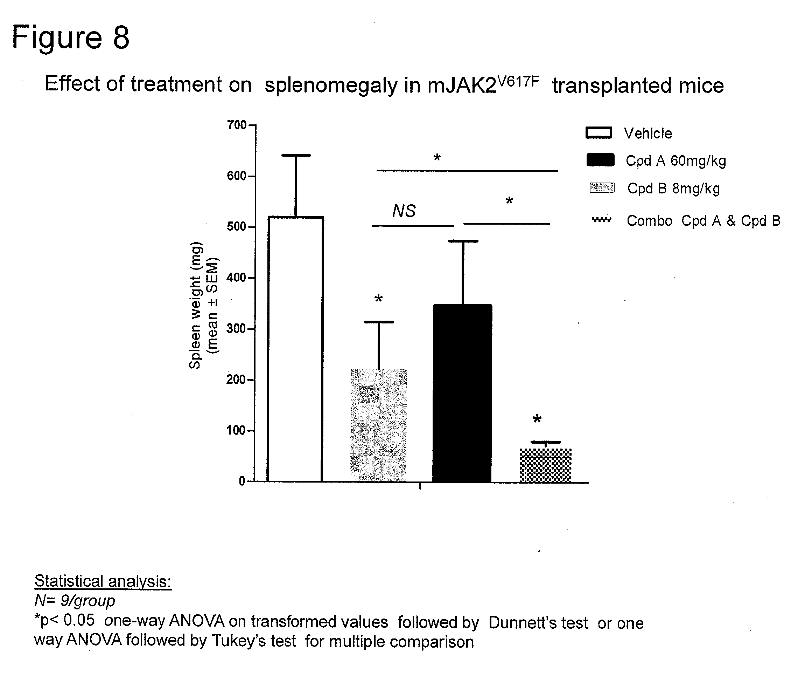

Figures 7 and 8 show Balb/c female mice transplanted with JAK2V617F bone marrow transduced cells received either vehicle, Compound B at a dose of 8 mg/kg i.p. (free-base equivalent) on a M/W/F schedule, Compound A at dose of 60 mg/kg (free-base equivalent) q12h or the combination of both agents for 21 consecutive days. Change in body weight and spleen weight at sacrifice are depicted as mean ± SEM. N = 9/group. The average value for Balb/c female normal spleen weight is in the range of 100 mg,).*p < 0.05 on sacrifice day (one way ANOVA one way followed by Dunnett's test or Tukey's test.The reciprocal form of the spleen weight values were used for statistical analysis).

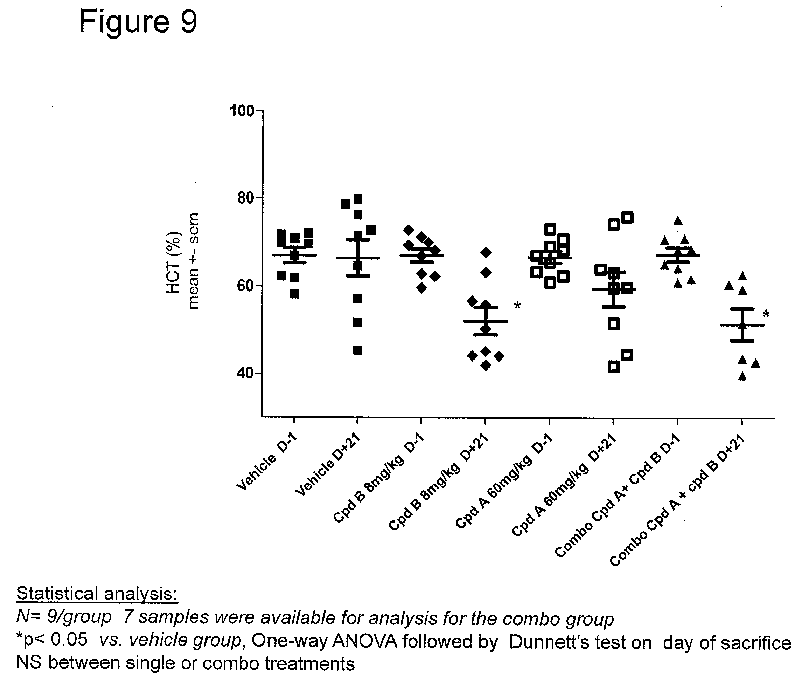

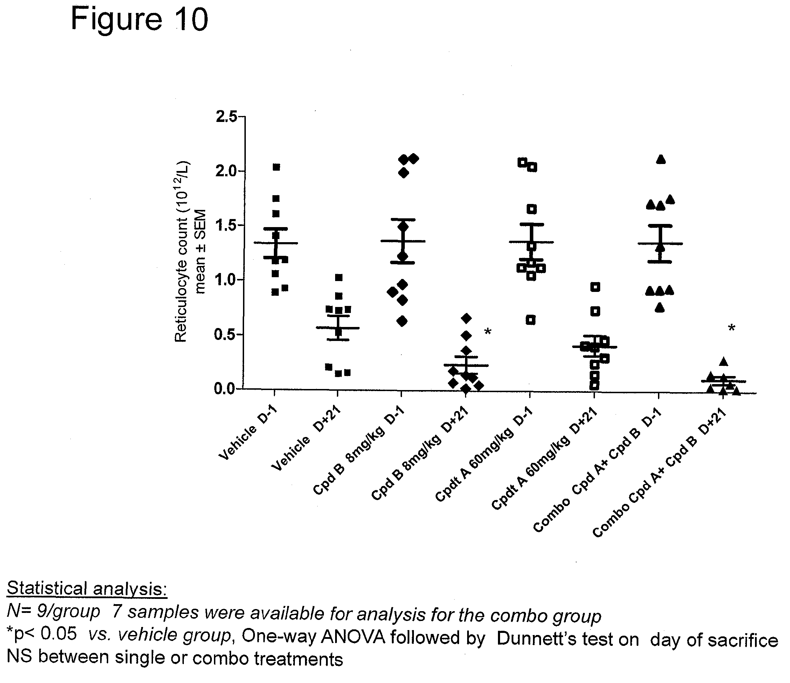

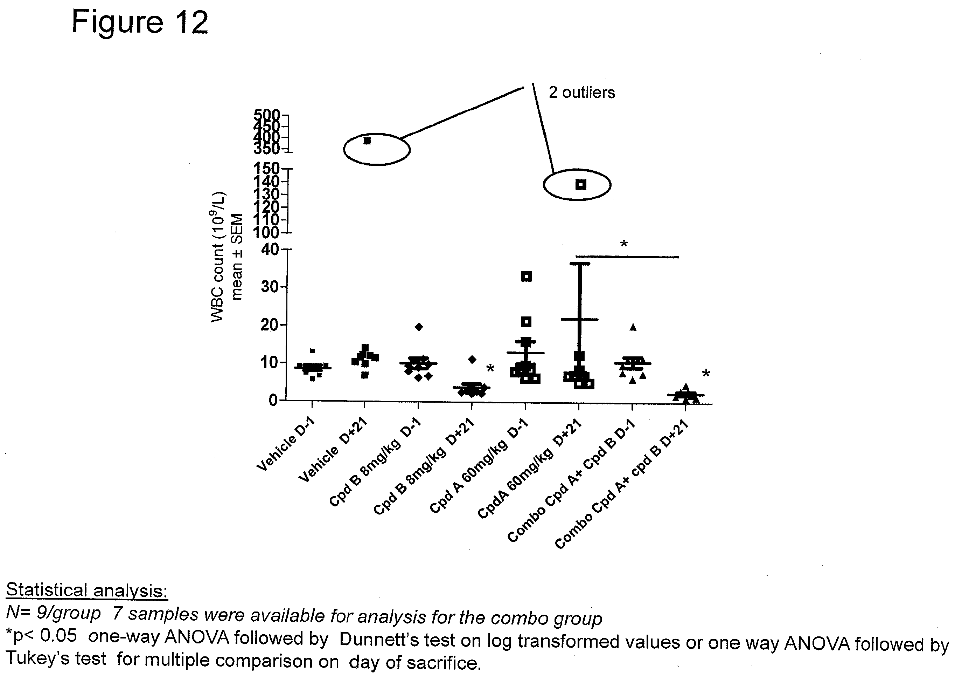

Figures 9-12 show Balb/c female mice transplanted with mJAK2V617F-IRES-GFP bone marrow transduced cells, which received either vehicles, Compound B at a dose of 8 mg/kg i.p. (free-base equivalent) on a M/W/F schedule, Compound A at a bid dose of 60 mg/kg (free-base equivalent) or the combination of both agents for 21 consecutive days. On day of sacrifice, blood was collected and analyzed with a Sysmex blood analyzer. N = 7-9 /group (some samples were not amenable to be analyzed in the Compound A/Compound B combo group. 1 outlier was detected in vehicle group (ID 3) and 1 outlier in Compound A group (ID 27) on sacrifice day based on analysis in Graph Pad Quick calcs outlier program (data analysis was performed with all values). Figure 9: Hct;
Figure 10: Reticulocyte count; Figure 11: Platelet count; Figure 12: White blood cell count. *p < 0.05 on sacrifice day (One way ANOVA followed by Dunnett's test or Tukey's test for multiple comparisons on log10 transformer values for WBC and PLT counts).
Figures 13-16 show representative bone marrow staining images of Balb/c female mice that were transplanted with mJAK2V617F-IRES-GFP cells and treated with Compound B at a dose of 8 mg/kg i.p. (free-base equivalent) on a M/W/F schedule, Compound A at dose of 60 mg/kg (free-base equivalent) q12h or the combination of both agents for 21 consecutive days.
Figures 17-19 show representative spleen staining images of Balb/c female mice that were transplanted with mJAK2V617F-IRES-GFP cells and treated with Compound B at a dose of 8 mg/kg i.p. (free-base equivalent) on a M/W/F schedule, Compound A at dose of 60 mg/kg (free-base equivalent) q12h or the combination of both agents for 21 consecutive days.
Figure 20 illustrates the study design and methods.
Figure 21 illustrates the palpable speen length over time in Cohort 1.
Figure 22 illustrates the palpable speen length over time in Cohort 2.
Figure 23 illustrates the palpable speen length over time in Cohort 3.

### Detailed Description

It has been discovered that administering a combination of
(a) Compound A ((R)-3-(4-(7H-pyrrolo[2,3-d]pyrimidin-4-yl)-1H-pyrazol-1-yl)-3-cyclopentylpropanenitrile) of Formula (A): 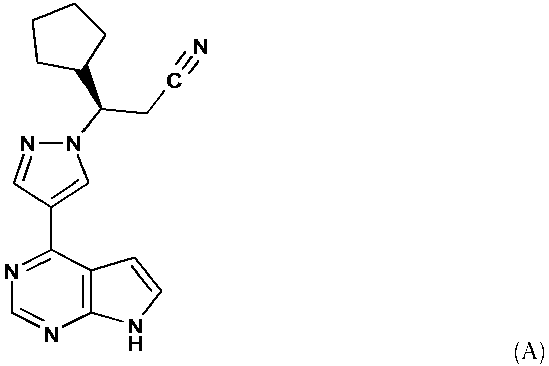 or a pharmaceutically acceptable salt thereof; and
(b) Compound B (*N*-hydroxy-3-[4-[[[2-(2-methyl-1*H*-indol-3-yl)-ethyl]-amino]methyl]phenyl]-2*E*-2-propenamide) of Formula (B): 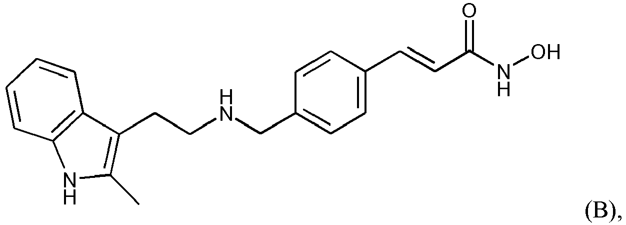 or a pharmaceutically acceptable salt thereof;
provides surprising, synergistic effects for treating cancer, in a subject. Such an approach - combination or co-administration of the two types of agents - can be useful for treating individuals suffering from cancer who do not respond to or are resistant to currently-available therapies. The combination therapy provided herein is also useful for improving the efficacy and/or reducing the side effects of currently-available cancer therapies for individuals who do respond to such therapies.

Certain terms used herein are described below. Compounds of the present invention are described using standard nomenclature. Unless defined otherwise, all technical and scientific terms used herein have the same meaning as is commonly understood by one of skill in the art to which this invention belongs.

Compound A and its pharmaceutically acceptable salt(s) thereof are described in the literature, for example, US Patent 7,598,257. Compound A is also known as ruxolitinib.

Compound B and its pharmaceutically acceptable salt(s) thereof are described in the literature, for example, US Patents 6,833,384; 7,067551; and 6,552,065. Compound B is also known as panobinostat.

Unless otherwise specified, or clearly indicated by the text, reference to compounds useful in the combination therapy of the invention includes both the free base of the compounds, and all pharmaceutically acceptable salts of the compounds.

As used herein, the term "pharmaceutically acceptable salts" refers to the nontoxic acid or alkaline earth metal salts of the pyrimidine compounds of the invention. These salts can be prepared *in situ* during the final isolation and purification of the pyrimidine compounds, or by separately reacting the base or acid functions with a suitable organic or inorganic acid or base, respectively. Representative salts include, but are not limited to, the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemi-sulfate, heptanoate, hexanoate, fumarate, hydrochloride, hydrobromide, hydroiodide, 2-hydroxyethanesulfonate, lactate, maleate, methanesulfonate, nicotinate, 2-naphthalenesulfonate, oxalate, pamoate, pectinate, persulfate, 3-phenylproionate, phosphate, picrate, pivalate, propionate, succinate, sulfate, tartrate, thiocyanate, p-toluenesulfonate, and undecanoate. Also, the basic nitrogen-containing groups can be quaternized with such agents as alkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl, and stearyl chlorides, bromides and iodides, aralkyl halides like benzyl and phenethyl bromides, and others. Water or oil-soluble or dispersible products are thereby obtained.

Examples of acids that may be employed to form pharmaceutically acceptable acid addition salts include such inorganic acids as hydrochloric acid, hydroboric acid, nitric acid, sulfuric acid and phosphoric acid and such organic acids as formic acid, acetic acid, trifluoroacetic acid, fumaric acid, tartaric acid, oxalic acid, maleic acid, methanesulfonic acid, succinic acid, malic acid, methanesulfonic acid, benzenesulfonic acid, and p-toluenesulfonic acid, citric acid, and acidic amino acids such as aspartic acid and glutamic acid.

Basic addition salts can be prepared *in situ* during the final isolation and purification of the pyrimidine compounds, or separately by reacting carboxylic acid moieties with a suitable base such as the hydroxide, carbonate or bicarbonate of a pharmaceutically acceptable metal cation or with ammonia, or an organic primary, secondary or tertiary amine. Pharmaceutically acceptable salts include, but are not limited to, cations based on the alkali and alkaline earth metals, such as sodium, lithium, potassium, calcium, magnesium, aluminum salts and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like. Other representative organic amines useful for the formation of base addition salts include diethylamine, ethylenediamine, ethanolamine, diethanolamine, piperazine, pyridine, picoline, triethanolamine and the like, and basic amino acids such as arginine, lysine and ornithine.

Provided herein is a combination for use in the therapy comprising a JAK inhibitor Compound A and HDAC inhibitor Compound B. Administration of the combination includes administration of the combination in a single formulation or unit dosage form, administration of the individual agents of the combination concurrently but separately, or administration of the individual agents of the combination sequentially by any suitable route. The dosage of the individual agents of the combination may require more frequent administration of one of the agent(s) as compared to the other agent(s) in the combination. Therefore, to permit appropriate dosing, packaged pharmaceutical products may contain one or more dosage forms that contain the combination of agents, and one or more dosage forms that contain one of the combination of agents, but not the other agent(s) of the combination.

The term "single formulation" as used herein refers to a single carrier or vehicle formulated to deliver effective amounts of both therapeutic agents to a patient. The single vehicle is designed to deliver an effective amount of each of the agents, along with any pharmaceutically acceptable carriers or excipients. In some embodiments, the vehicle is a tablet, capsule, pill, or a patch. In other embodiments, the vehicle is a solution or a suspension.

The term "unit dose" is used herein to mean simultaneous administration of both agents together, in one dosage form, to the patient being treated. In some embodiments, the unit dose is a single formulation. In certain embodiments, the unit dose includes one or more vehicles such that each vehicle includes an effective amount of at least one of the agents along with pharmaceutically acceptable carriers and excipients. In some embodiments, the unit dose is one or more tablets, capsules, pills, or patches administered to the patient at the same time.

The term "treat" is used herein to mean to relieve, reduce or alleviate, at least one symptom of a disease in a subject. Within the meaning of the present invention, the term "treat" also denotes, to arrest, delay the onset (*i.e.,* the period prior to clinical manifestation of a disease or symptom of a disease) and/or reduce the risk of developing or worsening a symptom of a disease.

The term "subject" is intended to include animals. Examples of subjects include mammals, *e.g*., humans, dogs, cows, horses, pigs, sheep, goats, cats, mice, rabbits, rats, and transgenic non-human animals. In certain embodiments, the subject is a human, *e.g*., a human suffering from, at risk of suffering from, or potentially capable of suffering from multiple myeloma.

The term "about" or "approximately" usually means within 20%, more preferably within 10%, and most preferably still within 5% of a given value or range. Alternatively, especially in biological systems, the term "about" means within about a log (*i.e.,* an order of magnitude) preferably within a factor of two of a given value.

The combination of agents described herein display a synergistic effect. The term "synergistic effect" as used herein, refers to action of two agents producing an effect, for example, slowing the symptomatic progression of cancer or symptoms thereof, which is greater than the simple addition of the effects of each drug administered by themselves. A synergistic effect can be calculated, for example, using suitable methods such as the Sigmoid-Emax equation (Holford, N. H. G. and Scheiner, L. B., Clin. Pharmacokinet. 6: 429-453 (1981)), the equation of Loewe additivity (Loewe, S. and Muischnek, H., Arch. Exp. Pathol Pharmacol. 114: 313-326 (1926)) and the median-effect equation (Chou, T. C. and Talalay, P., Adv. Enzyme Regul. 22: 27-55 (1984)). Each equation referred to above can be applied to experimental data to generate a corresponding graph to aid in assessing the effects of the drug combination. The corresponding graphs associated with the equations referred to above are the concentration-effect curve, isobologram curve and combination index curve, respectively.

An "effective amount" of a combination of agents is an amount sufficient to provide an observable improvement over the baseline clinically observable signs and symptoms of the depressive disorder treated with the combination.

An "oral dosage form" includes a unit dosage form prescribed or intended for oral administration.

### Compound A and Compound B Combination for use in the treatment

The invention provides a combination of Compound A and Compound B for use in treating JAK-associated diseases as defined in the claims , *e.g.,* cancer, *e.g.,* myeloproliferative neoplasms, in an individual by administering to the individual a combination of Compound A and Compound B.

In one embodiment, provided herein is a combination of the present invention or a pharmaceutical composition thereof for use in treating a claimed JAK-associated disease or disorder in a subject (e.g., patient), wherein a therapeutically effective amount or dose of the combination of the present invention or the pharmaceutical composition thereof is administered to the individual in need of such treatment. A JAK-associated disease can include any disease, disorder or condition that is directly or indirectly linked to expression or activity of the JAK, including over-expression and/or abnormal activity levels. A JAK-associated disease can also include any disease, disorder or condition that can be prevented, ameliorated, or cured by modulating JAK activity.

Examples of JAK-associated diseases include diseases involving the immune system including, for example, organ transplant rejection (e.g., allograft rejection and graft versus host disease).

Further examples of JAK-associated diseases include autoimmune diseases such as multiple sclerosis, rheumatoid arthritis, juvenile arthritis, type I diabetes, lupus, psoriasis, inflammatory bowel disease, ulcerative colitis, Crohn's disease, myasthenia gravis, immunoglobulin nephropathies, autoimmune thyroid disorders, and the like. In some embodiments, the autoimmune disease is an autoimmune bullous skin disorder such as pemphigus vulgaris (PV) or bullous pemphigoid (BP).

Further examples of JAK-associated diseases include allergic conditions such as asthma, food allergies, atopic dermatitis and rhinitis. Further examples of JAK-associated diseases include viral diseases such as Epstein Barr Virus (EBV), Hepatitis B, Hepatitis C, HIV, HTLV 1, Varicella-Zoster Virus (VZV) and Human Papilloma Virus (HPV).

Further examples of JAK-associated diseases or conditions include skin disorders such as psoriasis (for example, psoriasis vulgaris), atopic dermatitis, skin rash, skin irritation, skin sensitization (e.g., contact dermatitis or allergic contact dermatitis). For example, certain substances including some pharmaceuticals when topically applied can cause skin sensitization. In some embodiments, the skin disorder is treated by topical administration of at least one JAK inhibitor of the invention.

In further embodiments, the JAK-associated disease is cancer including those characterized by solid tumors (e.g., prostate cancer, renal cancer, hepatic cancer, pancreatic cancer, gastric cancer, breast cancer, lung cancer, cancers of the head and neck, thyroid cancer, glioblastoma, Kaposi's sarcoma, Castleman's disease, melanoma *etc*.), hematological cancers (e.g., lymphoma, leukemia such as acute lymphoblastic leukemia, or multiple myeloma), and skin cancer such as cutaneous T-cell lymphoma (CTCL) and cutaneous B-cell lymphoma. Example cutaneous T-cell lymphomas include Sezary syndrome and mycosis fungoides.

JAK-associated diseases can further include those characterized by expression of a mutant JAK2 such as those having at least one mutation in the pseudo-kinase domain (e.g., JAK2V617F).

JAK-associated diseases can further include myeloproliferative disorders (MPDs) such as polycythemia vera (PV), essential thrombocythemia (ET), myeloid metaplasia with myelofibrosis (MMM), chronic myelogenous leukemia (CML), chronic myelomonocytic leukemia (CMML), hypereosinophilic syndrome (HES), systemic mast cell disease (SMCD), and the like.

Further JAK-associated diseases include inflammation and inflammatory diseases. Example inflammatory diseases include inflammatory diseases of the eye (e.g., iritis, uveitis, scleritis, conjunctivitis, or related disease), inflammatory diseases of the respiratory tract (e.g., the upper respiratory tract including the nose and sinuses such as rhinitis or sinusitis or the lower respiratory tract including bronchitis, chronic obstructive pulmonary disease, and the like), inflammatory myopathy such as myocarditis, and other inflammatory diseases.

The combination described herein can further be for use in the treatment of ischemia reperfusion injuries or a disease or condition related to an inflammatory ischemic event such as stroke or cardiac arrest. The combination described herein can further be for use in the treatment of anorexia, cachexia, or fatigue such as that resulting from or associated with cancer. The combination described herein can further be for use in the treatment of restenosis, sclerodermitis, or fibrosis. The combination described herein can further be for use in the treatment of conditions associated with hypoxia or astrogliosis such as, for example, diabetic retinopathy, cancer, or neurodegeneration.

Provided herein are Compound A and Compound B for use in the methods of treating disease, *e.g.,* a myeloproliferative disorder, by administering an effective amount of Compound A and Compound B to an individual suffering from a disease. The amount of the combination of agents is effective to treat the disease. It is important to note the synergistic effects of the combination of agents: even though one or more of the agents administered alone at a particular dosage may not be effective, when administered in combination, at the same dosage of each agent, the treatment is effective. The doses of the one or more of the agents in the combination therefore can be less than the FDA approved doses of each agent.

### Dosages

The optimal dose of the combination of agents for use in the treatment of disease can be determined empirically for each individual using known methods and will depend upon a variety of factors, including, though not limited to, the degree of advancement of the disease; the age, body weight, general health, gender and diet of the individual; the time and route of administration; and other medications the individual is taking. Optimal dosages may be established using routine testing and procedures that are well known in the art.

The amount of combination of agents that may be combined with the carrier materials to produce a single dosage form will vary depending upon the individual treated and the particular mode of administration. In some embodiments the unit dosage forms containing the combination of agents as described herein will contain the amounts of each agent of the combination that are typically administered when the agents are administered alone.

Frequency of dosage may vary depending on the compound used and the particular condition to be treated or prevented. In general, the use of the minimum dosage that is sufficient to provide effective therapy is preferred. Patients may generally be monitored for therapeutic effectiveness using assays suitable for the condition being treated or prevented, which will be familiar to those of ordinary skill in the art.

The dosage form can be prepared by various conventional mixing, comminution and fabrication techniques readily apparent to those skilled in the chemistry of drug formulations.

The oral dosage form containing the combination of agents or individual agents of the combination of agents may be in the form of micro-tablets enclosed inside a capsule, *e.g.* a gelatin capsule. For this, a gelatin capsule as is employed in pharmaceutical formulations can be used, such as the hard gelatin capsule known as CAPSUGEL, available from Pfizer.

Many of the oral dosage forms useful herein contain the combination of agents or individual agents of the combination of agents in the form of particles. Such particles may be compressed into a tablet, present in a core element of a coated dosage form, such as a taste-masked dosage form, a press coated dosage form, or an enteric coated dosage form, or may be contained in a capsule, osmotic pump dosage form, or other dosage form.

The drug compounds of the present invention are present in the combinations, dosage forms, pharmaceutical compositions and pharmaceutical formulations disclosed herein in a ratio in the range of 100:1 to 1:100, more preferably 1:1 to 1:100, and still more preferably 1:10 to 1:100.

In one embodiment, when adiministered to a human, Ruxolitinib is given 5 mg BID, Panobinostat is administered 10 mg TIW QOW.

In one embodiment, when adiministered to a human, Ruxolitinib is given 10 mg BID, Panobinostat is administered 10 mg TIW QOW.

In one embodiment, when adiministered to a human, Ruxolitinib is given 15 mg BID, Panobinostat is administered 10 mg TIW QOW.

In one embodiment, when adiministered to a human, Ruxolitinib is given 15 mg BID, Panobinostat is administered 15 mg TIW QOW.

In one embodiment, when adiministered to a human, Ruxolitinib is given 15 mg BID, Panobinostat is administered 20 mg TIW QOW.

In one embodiment, when adiministered to a human, Ruxolitinib is given 15 mg BID, Panobinostat is administered 25 mg TIW QOW.

In one embodiment, when adiministered to a human, Ruxolitinib is given 15 mg BID, Panobinostat is administered 30 mg TIW QOW.

In one embodiment, when adiministered to a human, Ruxolitinib is given 3-7 mg BID, Panobinostat is administered 8-12 mg TIW QOW.

In one embodiment, when adiministered to a human, Ruxolitinib is given 8-12 mg BID, Panobinostat is administered 8-12 mg TIW QOW.

In one embodiment, when adiministered to a human, Ruxolitinib is given 10-20 mg BID, Panobinostat is administered 8-12 mg TIW QOW.

In one embodiment, when adiministered to a human, Ruxolitinib is given 10-20 mg BID, Panobinostat is administered 10-20 mg TIW QOW.

In one embodiment, when adiministered to a human, Ruxolitinib is given 10-20 mg BID, Panobinostat is administered 10-30 mg TIW QOW.

In one embodiment, when adiministered to a human, Ruxolitinib is given 10-20 mg BID, Panobinostat is administered 10-30 mg TIW QOW.

In one embodiment, when adiministered to a human, Ruxolitinib is given 10-20 mg BID, Panobinostat is administered 20-40 mg TIW QOW.

The optimum ratios, individual and combined dosages, and concentrations of the drug compounds that yield efficacy without toxicity are based on the kinetics of the active ingredients' availability to target sites, and are determined using methods known to those of skill in the art.

### Pharmaceutical Compositions

The pharmaceutical compositions or combinations provided herein can be tested in clinical studies. Suitable clinical studies may be, for example, open label, dose escalation studies in patients with proliferative diseases. Such studies prove in particular the synergism of the active ingredients of the combination of the invention. The beneficial effects on proliferative diseases may be determined directly through the results of these studies which are known as such to a person skilled in the art. Such studies may be, in particular, be suitable to compare the effects of a monotherapy using the active ingredients and a combination of the invention.

The administration of a pharmaceutical combination of the invention may result not only in a beneficial effect, *e.g.* a synergistic therapeutic effect, *e.g.* with regard to alleviating, delaying progression of or inhibiting the symptoms, but also in further surprising beneficial effects, *e.g.* fewer side-effects, an improved quality of life or a decreased morbidity, compared with a monotherapy applying only one of the pharmaceutically active ingredients used in the combination of the invention.

A further benefit may be that lower doses of the active ingredients of the combination of the invention may be used, for example, that the dosages need not only often be smaller but may also be applied less frequently, which may diminish the incidence or severity of side-effects. This is in accordance with the desires and requirements of the patients to be treated.

It is one objective of this invention to provide a pharmaceutical composition comprising a quantity, which may be jointly therapeutically effective at targeting or preventing cancer, *e.g.,* a myeloproliferative disorder. In this composition, Compound A and Compound B may be administered together, one after the other or separately in one combined unit dosage form or in two separate unit dosage forms. The unit dosage form may also be a fixed combination.

The pharmaceutical compositions for separate administration of both compounds, or for the administration in a fixed combination, *i.e.* a single galenical composition comprising both compounds according to the invention may be prepared in a manner known *per se* and are those suitable for enteral, such as oral or rectal, and parenteral administration to mammals (warm-blooded animals), including humans, comprising a therapeutically effective amount of at least one pharmacologically active combination partner alone, *e.g.* as indicated above, or in combination with one or more pharmaceutically acceptable carriers or diluents, especially suitable for enteral or parenteral application.

### Formulations

The drug combinations provided herein may be formulated by a variety of methods apparent to those of skill in the art of pharmaceutical formulation. The various release properties described above may be achieved in a variety of different ways. Suitable formulations include, for example, tablets, capsules, press coat formulations, and other easily administered formulations.

Suitable pharmaceutical formulations may contain, for example, from about 0.1 % to about 99.9%, preferably from about 1 % to about 60 %, of the active ingredient(s). Pharmaceutical formulations for the combination therapy for enteral or parenteral administration are, for example, those in unit dosage forms, such as sugar-coated tablets, tablets, capsules or suppositories, or ampoules. If not indicated otherwise, these are prepared in a manner known *per se,* for example by means of conventional mixing, granulating, sugar-coating, dissolving or lyophilizing processes. It will be appreciated that the unit content of a combination partner contained in an individual dose of each dosage form need not in itself constitute an effective amount since the necessary effective amount may be reached by administration of a plurality of dosage units.

In particular, a therapeutically effective amount of each of the combination partner of the combination of the invention may be administered simultaneously or sequentially and in any order, and the components may be administered separately or as a fixed combination. For example, the combination for use in treating a disease according to the invention may comprise (i) administration of the first agent (a) in free or pharmaceutically acceptable salt form and (ii) administration of an agent (b) in free or pharmaceutically acceptable salt form, simultaneously or sequentially in any order, in jointly therapeutically effective amounts, preferably in synergistically effective amounts, *e.g.* in daily or intermittently dosages corresponding to the amounts described herein. The individual combination partners of the combination of the invention may be administered separately at different times during the course of therapy or concurrently in divided or single combination forms. The instant invention is therefore to be understood as embracing all such regimens of simultaneous or alternating treatment and the term "administering" is to be interpreted accordingly.

The effective dosage of each of the combination partners employed in the combination of the invention may vary depending on the particular compound or pharmaceutical composition employed, the mode of administration, the condition being treated, the severity of the condition being treated. Thus, the dosage regimen of the combination of the invention is selected in accordance with a variety of factors including the route of administration and the renal and hepatic function of the patient. A clinician or physician of ordinary skill can readily determine and prescribe the effective amount of the single active ingredients required to alleviate, counter or arrest the progress of the condition.

### Examples

The present disclosure is further illustrated by the following non-limiting examples.

### EXAMPLE A

A study is conducted to test the efficacy of the combination of Compound A and Compound B in Scid-beige mice injected i.v. (tail vein) with 1·10⁶ Ba/F3 EpoR JAK2V617F-luc clone 8 cells.

### Protocol:

### Animals:

80 Scid-beige female mice, 18-22g (8 groups of 7 mice, 24 excluded at time of randomization)
Cells: Ba/F3 EpoR JAK2V617F-luc clone 8 cells.

Whole body Xenogen imaging was performed on days 4, 7, 9 and 11 after cell injection. Randomization of animals into treatment groups was based on bioluminescence (BioL) on day 4 after cell injection.

The mouse model and the protocol is described by Baffert et al., Mol. Cancer Ther; 9(7), 1945, July 2010.

### Animal and Cell line:

| | |
|---|---|
| Cell line: | Ba/F3EpoR JAK2V617F-luc clone 8 |
| Gene: | *JAK2*V617F |
| Cell-Nr. / Injection site: | 1x10e6cells in 200ul / tail i.v. injection |
| Species: | mouse |
| Strain: | CB17/C.B-Igh-1^{b}/GbmsTac-Prkdc^{scid}-lyst^{bg}N7, Taconic farms (complete name Taconic) |
| Gender: | female |
| Age: | 22-24 g |
| Number: | 80 mice |

### Cells preparation and injection:

| | |
|---|---|
| Reagents: | RPMI1640 (Amined) |
| | 10% FCS (Amined) |
| | L-Glutamine 2mM (Amined) |
| | Puromycin, 1ug/ml (Sigma, lot 036K4073) |
| | Hygromicin B, 100ug/ml (invitrogen, lot B13871010) |

Ba/F3 EpoR JAK2V617F-luc cells are grown in RPMI1640 supplemented with 10% FCS, 1% Glutamine, Puromycin (1ug/mL) and Hygromycin (100ug/mL). Cells are splited every three days (1:50) not more than two weeks before experiment. The day prior the injection, cells are grown without any antibiotics (to avoid any potential delay in cell growth in vivo).

The day of injection the cells are harvested and resuspended in HBSS buffer (10e6 cells in 200ul). Cells are injected in tail vein using 27G tuberculin syringe.

### Group definition:

Group 1 (7 mice): vehicle group (0.5% HPMC po + D5W 5% ip)
Group 2 (7 mice): Compound B 5 mg/kg , 3 times a week (M/W/F), ip + HPMC 0.5%po
Group 3 (7 mice): Compound B 10mg/kg, 3 times a week (M/W/F), ip + HPMC 0.5% po
Group 4 (7 mice): Compound B 15mg/kg, 3 times a week (M/W/F), ip + HPMC 0.5% po
Group 5 (7 mice): Compound A 79.2 mg/kg twice a day, po + D5W5% ip
Group 6 (7 mice): Compound A 79.2 mg/kg twice a day, po + Compound B 5mg/kg, 3 times a week (M/W/F), ip
Group 7 (7 mice): Compound A 79.2 mg/kg twice a day, po + Compound B 10mg/kg, 3 times a week (M/W/F), ip
Group 8 (7 mice): Compound A 79.2 mg/kg twice a day, po + Compound B 15mg/kg, 3 times a week (M/W/F), ip

Treatments are given simultaneously.

### Compounds:

Compound A is a monophosphate salt, 79.2 mg/kg (equivalent 60mg/kg free base), 10mL/kg in 0.5% HPMC Pharmacoat 603.

Formulation containing Compound A: 522.7mg Compound A (in monophosphate salt form) in 66ml HPMC Pharmacoat 603 0.5%.

Compound B is in lactic acid salt form, 15mg/kg (equivalent 11.90 mg/kg free base), 10mg/kg (equivalent 7.94 mg/kg free base) and 5mg/kg (equivalent 3.97 mg/kg free base), 10mL/kg in D5W 5% (B. Braun, Lot 395147).

Formulation containing Compound B: 15mg Compound B (in lactic acid salt form) in 10ml of D5W 5%, 10mg in 10ml of D5W 5% and 5mg in 10ml of D5W 5% respectively.

### Monitoring and data/sample collection:

Animals are identified by transponders and monitored daily.

Bioluminescence are recorded using the Xenogen Camera (non invasive IVIS^{®} Imaging system, Caliper) on day 4, day 7 and 10 post cell injection under isofluran anesthesia (3-5 % vol isofluran, 0.8 L /min air flow). Animals are injected i.p. with D-luciferin (Xenogen corporation, ref XR-1001), at 150mg/kg (10ml/kg, Nacl). Anesthetized mice are then placed in the imaging chamber for imaging 15 minutes after the luciferin injection.

Xenogen parameters: D/10sec/med/15min.

Animals are randomized on day 4 and treatment started for at least 6 days based on well being of vehicle treated animals.

Body weight (BW) and well being are recorded in score sheet. Animals are sacrificed if BW exceeds 15 % on 2 consecutive days. All mice are sacrificed if more than 50 % of the animals per group are dead.

### Sacrifice, samples collection:

2 hours after last dose for Compound A;
2 hours after last dose for Compound B;
   - Spleen weight is recorded at sacrifice.
   - Samples collected and snap frozen in liquid nitrogen and then kept at -80°C until analysis:
      - spleen, BM, blood and liver for PK analysis
      - spleen for PD marker analysis (p-STAT5 (Compound A) and protein acetylation (Compound B)),
      - sternum for PD marker analysis (p-STAT5 (Compound A).

### Results of Example A:

Doses expressed as free-base equivalent.

Tables 1.1-1.8 show the Bioluminescence at time of randomization for the different groups.

**Table 1.1**

| **Group 1 Vehicle** | **Mouse nb** | **BioL day 4** |
|---|---|---|
| 24 | 1420 | 8.47E+05 |
| 21 | 2009 | 1.07E+06 |
| 70 | 2057 | 1.08E+06 |
| 76 | 6080 | 1.43E+06 |
| 75 | 7426 | 1.46E+06 |
| 29 | 4546 | 1.98E+06 |
| 6 | 7540 | 2.04E+06 |
| | | |
| **Average** | | **1.42E+06** |
| **Stdev** | | **4.60E+05** |
| **Sem** | | **1.74E+05** |

**Table 1.2**

| **Group 2 Compound B 4 mg/kg** | **Mouse nb** | **BioL day4** |
|---|---|---|
| 47 | 1367 | 8.51E+05 |
| 22 | 2330 | 1.06E+06 |
| 58 | 4827 | 1.10E+06 |
| 26 | 8199 | 1.40E+06 |
| 61 | 5411 | 1.47E+06 |
| 17 | 6505 | 1.89E+06 |
| 62 | 3364 | 2.05E+06 |
| | | |
| **Average** | | **1**.**40E**+**06** |
| **Stdev** | | **4**.**42E**+**05** |
| **Sem** | | **1**.**67E**+**05** |

**Table 1.3**

| **Group 3 Compound B 8 mg/kg** | **Mouse nb** | **BioL day4** |
|---|---|---|
| 19 | 313 | 8.52E+05 |
| 45 | 4578 | 1.06E+06 |
| 23 | 4191 | 1.10E+06 |
| 72 | 6127 | 1.39E+06 |
| 31 | 3335 | 1.50E+06 |
| 51 | 4942 | 1.86E+06 |
| 28 | 2809 | 2.06E+06 |
| | | |
| **Average** | | **1**.**40E**+**06** |
| **Stdev** | | **4**.**40E**+**05** |
| **Sem** | | **1**.**66E**+**05** |

**Table 1.4**

| **Group 4 Compound B 12 mg/kg** | **Mouse nb** | **BioL day4** |
|---|---|---|
| | | |
| 37 | 4716 | 8.69E+05 |
| 9 | 2896 | 1.02E+06 |
| 42 | 3748 | 1.10E+06 |
| 39 | 2934 | 1.37E+06 |
| 2 | 8255 | 1.51E+06 |
| 52 | 1681 | 1.81E+06 |
| 8 | 5002 | 2.10E+06 |
| | | |
| **Average** | | **1**.**40E**+**06** |
| **Stdev** | | **4.45E+05** |
| **Sem** | | **1**.**68E**+**05** |

**Table 1.5**

| **Group 5** | **Mouse nb** | **BioL day4** |
|---|---|---|
| **Compound A 60 mg/kg** | | |
| 40 | 6132 | 8.80E+05 |
| 66 | 870 | 9.78E+05 |
| 18 | 5782 | 1.11E+06 |
| 69 | 5545 | 1.35E+06 |
| 48 | 9104 | 1.55E+06 |
| 71 | 3957 | 1.74E+06 |
| 14 | 8439 | 2.24E+06 |
| | | |
| **Average** | | **1**.**41E**+**06** |
| **Stdev** | | **4**.**79E**+**05** |
| **Sem** | | **1**.**81E**+**05** |

**Table 1.6**

| **Group 6** | **Mouse nb** | **BioL day4** |
|---|---|---|
| **Compound A 60 mg/kg** | | |
| **Compound B 4 mg/kg** | | |
| 63 | 4010 | 9.04E+05 |
| 13 | 2805 | 9.70E+05 |
| 11 | 3830 | 1.13E+06 |
| 54 | 6791 | 1.26E+06 |
| 55 | 6962 | 1.59E+06 |
| 12 | 3656 | 1.71E+06 |
| 15 | 2875 | 2.31E+06 |
| | | |
| **Average** | | **1**.**41E**+**06** |
| **Stdev** | | **4**.**97E**+**05** |
| **Sem** | | **1**.**88E**+**05** |

**Table 1.7**

| **Group 7** | **Mouse nb** | **BioL day4** |
|---|---|---|
| **Compound A 60 mg/kg** | | |
| **Compound B 8 mg/kg** | | |
| 27 | 3824 | 9.06E+05 |
| 65 | 5471 | 9.62E+05 |
| 49 | 2321 | 1.14E+06 |
| 57 | 1127 | 1.22E+06 |
| 30 | 6769 | 1.60E+06 |
| 32 | 2622 | 1.71E+06 |
| 79 | 1594 | 2.46E+06 |
| | | |
| **Average** | | **1**.**43E**+**06** |
| **Stdev** | | **5**.**47E**+**05** |
| **Sem** | | **2**.**07E**+**05** |

**Table 1.8**

| **Group 8** | **Mouse nb** | **BioL day4** |
|---|---|---|
| **Compoun A 60mg/kg** | | |
| **Compound B 12mg/kg** | | |
| 41 | 2798 | 9.07E+05 |
| 33 | 7925 | 9.51E+05 |
| 73 | 5754 | 1.16E+06 |
| 38 | 2515 | 1.19E+06 |
| 77 | 8809 | 1.65E+06 |
| 20 | 6583 | 1.70E+06 |
| 7 | 5799 | 2.82E+06 |
| | | |
| **Average** | | **1**.**48E**+**06** |
| **Stdev** | | **6**.**67E**+**05** |
| **Sem** | | **2**.**52E**+**05** |

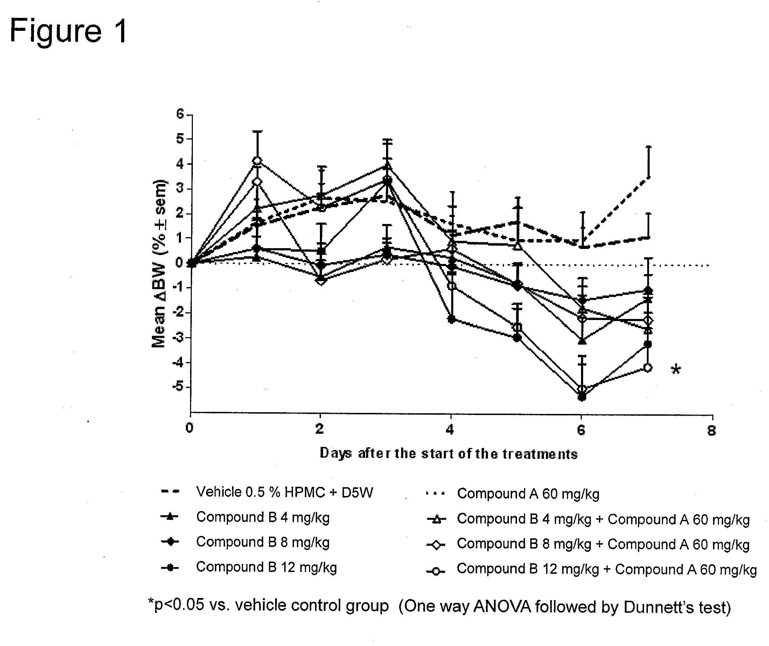

Figure 1 shows the body weight over the Compound A and/or Compound treatment period. There is no major change in tolerability when Compound A and Compound B are combined.

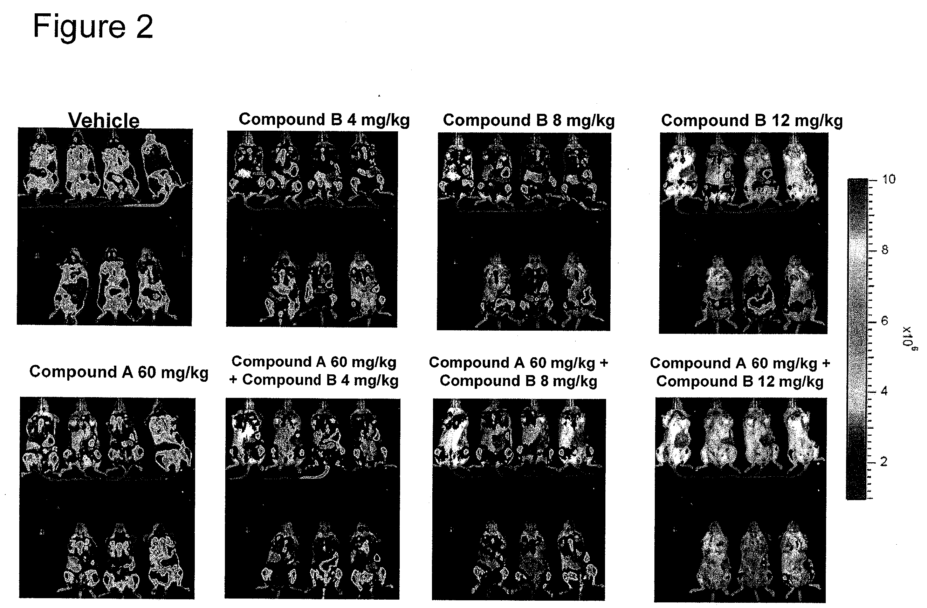

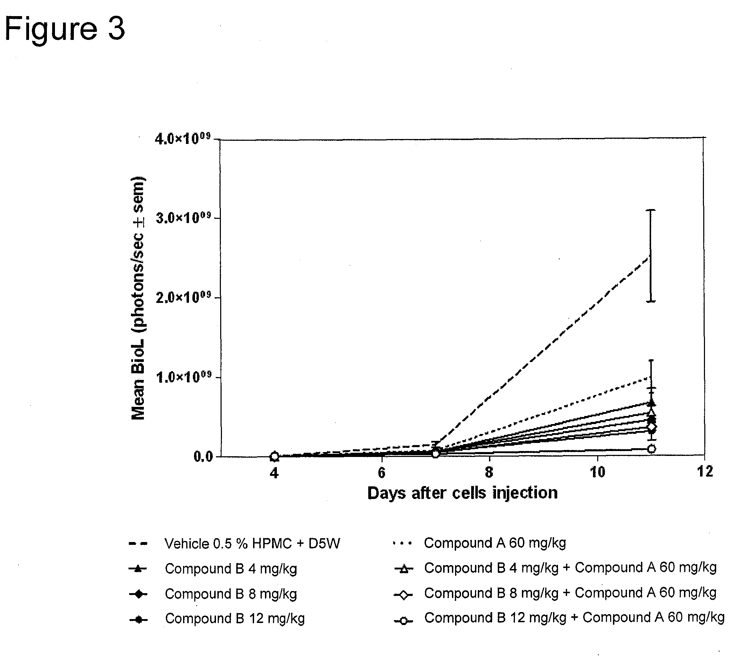

Figures 2 and 3 show the effect of treatment of BioL levels after 7 treatment days, i.e., day 11 after cell injection (treatment started on day 4 post cell injection).

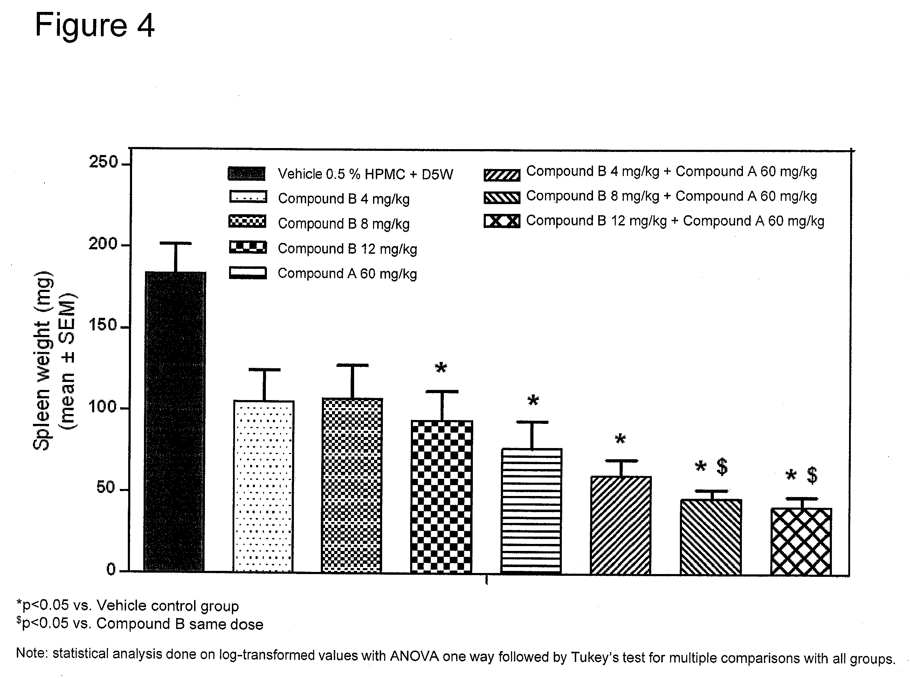

Figure 4 shows the spleen weight after 8 days of treatment.

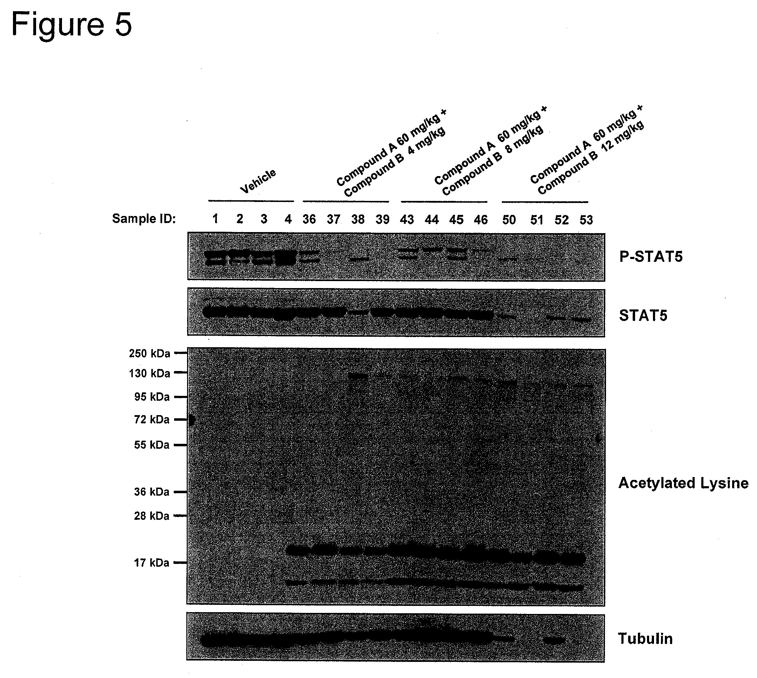

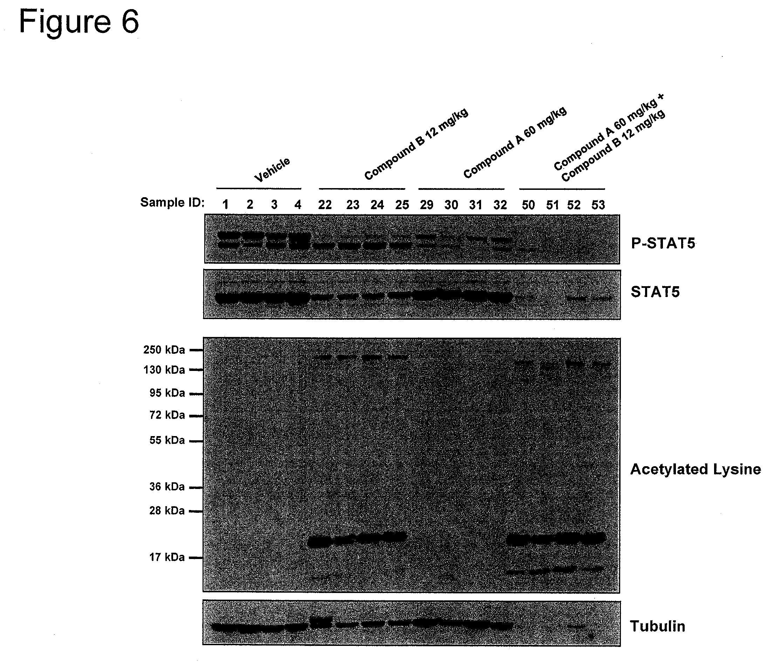

Figures 5 and 6 show the result of PD-marker analysis (spleen extracts) 2 hours post therapy.

Table 2 shows the effect of treatment on fractional increase in Biolux.

**Table 2.**

| Group | Vehicle | Compound B 4mg/kg | Compound B 8mg/kg | Compound B 12mg/kg | Compound A 60mg/kg | Compound A 60mg/kg + Compound B 4mg/kg | Compound A 60mg/kg + Compound B 8mg/kg | Compound A 60mg/kg + Compound B 12mg/kg |
|---|---|---|---|---|---|---|---|---|
| Fractional change in Biolux | 1767 ± 279 | 481 ± 83* | 351 ± 48* | 204 ± 51^{#}* | 717 ± 137* | 388 ± 48* | 267 ± 31^{#}* | 52 ± 9*^{#$} |
| T/C last day (%) | 100 | 27 | 20 | 11 | 40 | 22 ^{¥} | 15⁺ | 3^{&} |

| | | | | | | | | |
|---|---|---|---|---|---|---|---|---|
| **Notes:** Fractional change is calculated as: biolux on last day / biolux on first day. *, p<0.05 vs. vehicle control group ^{#}, p<0.05 vs. Compound A ^{$}, p<0.05 vs. Conmpound B same dose (ANOVA one way followed by Tukey's test on log transformed values for multiple comparisons with all groups) T/C calculated as: (treated/control)·100 According to the method described in Clarke R, Breast Cancer Res Treat, 1997: ^{¥}, slight antagonism is indicated ⁺, slight antagonism or additivity is indicated ^{&}, slight synergy or additivity is indicated | | | | | | | | |

**Table 3 lists the results of PK analysis 2 hours post-therapy.**

| | **Blood mmol/L** | | **Spleen nmol/g** | | **Liver nmol/g** | | **BM* nmol/g** | |
|---|---|---|---|---|---|---|---|---|
| Treatment | Compound A | Compound B | Compound A | Compound B | Compound A | Compound B | Compound A | Compound B |
| Compound B 4 mg/kg 2 hr | - | BLQ | - | 1.40 ± 0.19 | - | 0.18 ± 0.02 | - | 3.34 ± 0.35 |
| Compound B 8 mg/kg 2 hr | - | BLQ | - | 2.07 ± 0.20 | - | 0.25 ± 0.03 | - | 3.17 ± 0.36 |
| Compound B 12 mg/kg 2 hr | - | BLQ | - | 3.64 ± 0.53 | - | 0.63 ± 0.13 | - | 7.32 ± 1.44 |
| Compound A 60 mg/kg 2 hr | 1.96 ± 0.33 | - | 1.28 ± 0.28 | - | 4.54 ± 1.38 | - | 1.40 ± 0.65 | - |
| Compound A 60 mg/kg Compound B **4** mg/kg 2 hr | 2.78 ± 0.46 | 0.02 ± 0.01 | 1.15 ± 0.19 | 2.80 ± 0.37 | 3.49 ± 0.54 | 0.30 ± 0.05 | 2.59 ± 0.61 | 6.15 ± 0.61 |
| Compound A 60 mg/kg Compound B **8** mg/kg 2 hr | 2.23 ± 0.35 | 0.03 ± 0.00 | 1.02 ± 0.19 | 3.02 ± 0.25 | 2.91 ± 0.40 | 0.32 ± 0.02 | 1.92 ± 0.33 | 8.43 ± 0.94 |
| Compound A 60 mg/kg Compound B **12** mg/kg 2 hr | 2.72 ± 0.58 | 0.02 ± 0.01 | 1.61 ± 0.44 | 4.51 ± 0.57 | 3.87 ± 0.99 | 0.71 ± 0.03 | 2.29 ± 0.29 | 9.81 ± 1.17 |

| | | | | | | | | |
|---|---|---|---|---|---|---|---|---|
| All doses are given as free-base equivalent. BLQ: Below limit of quantification. Results depicted as mean SEM, *BM levels to be taken with caution due to issues with chromatogram. | | | | | | | | |

The results above indicate that the combination of Compound A and Compound B is tolerated in a mechanistic mouse model of Ba/F3 EpoR JAK2V617F-luc cells-driven leukemic disease (body weight loss comparable between Compound B mono-therapy groups and Compound B + Compound A combination groups).

When assessing leukemic cell spreading (Xenogen Biolux readout), the combination of Compound A and Compound B at doses of 60 mg/kg and 12 mg/kg, respectively, shows a significant difference as compared to these drugs given as mono-therapy.

### EXAMPLE B

The activity and tolerability of the deacetylase inhibitor Compound B in combination with the JAK1/2 inhibitor, Compound A, was evaluated in the JAK2V617F bone marrow transplant model of polycythemia vera-like disease. The combination activity in this MPN disease model was tested at doses of 8 mg/kg Compound B and 60 mg/kg Compound A. As shown in detail below, the combination of Compound B with the JAK1/2 inhibitor, Compound A, showed significant improvement on splenomegaly and bone marrow and spleen histology compared to each single agent.

### Bone marrow transplantation and analysis

### Bone marrow infection and transplantation

Murine bone marrow transplant experiments were performed essentially as described in Wernig et al. (Wernig, G., Mercher, T., OkabeR., et al. (2006) Expression of JAK2V617F causes a polycythemia vera-like disease with associated myelofibrosis in a murine bone marrow transplant model. Blood 107: 4274-4281). Briefly, Balb/c donor mice (6-8 weeks, Charles River, males or females) were treated 5 days prior BM transplant with 5-fluorouracil (150 mg/kg, i.p., Sigma-Aldrich, cat # F6627). Bone marrow cells from donor mice were harvested by flushing femurs and tibias. After red cell lysis (StemCellTechnologies, Grenoble, France, cat # 07800), nucleated cells were cultured for 24 hours in transplant medium (RPMI + 10 % FCS + 25 ng/ml IL3 (R&D Systems, Abington, UK, cat # 403-ML), 25 ng/ml IL6 (R&D Systems cat # 406-ML), and 50 ng/ml Stem Cell Factor (SC F, Bioconcept, Allschwill, Switzerland, cat # D-63120). The next day, cells were plated at 2 x 10⁶cells/ml/well in 6 well plates in the presence of 1 mL of supernatant containing virions from 293T transfected with 10 µg of p-MSCV-JAK2V617F-IRES-GFP vector (modification of original vector p-MSCV vector purchased from Clontech) and 10 µg of pCL-ECO vector (Imgenex, cat #10045P). After two spin-infections (2500 rpm for 90 min at 32°C, Multifuge 1S-R, Heraeus) unselected infected BM cells were resuspended in Hank's balanced salt solution (HBSS) before cell injection (1- 3x10⁶ depending on infection rate) into the tail vein of lethally irradiated Balb/c female recipient mice (total 9 Gy or 7 Gy) given as two 4.5 or 3.5 Gy doses 4 hours apart) using a BIOBEAM 8000, gamma irradiator (BEBIG GmbH, Germany).

Animals were monitored daily and sacrificed in case of major signs of distress (excessive body weight loss over more than 2 consecutive days without improvement, combined with lethargy and piloerection).

### Collection of samples and analysis

### CBC and organ collection

Blood was drawn from the tail vein or at sacrifice from the vena cava with a 20 G needle under isoflurane anesthesia (terminal procedure) and analyzed using an automated complete and differential blood cell counter (capillary mode using dilution 1:5 on a Sysmex blood analyzer, XT2000iV, Sysmex Digitana AG, Norderstedt, Germany). Spleens were weighed to evaluate splenomegaly. Blood, spleens, sterna, and livers were collected for pharmacokinetic, pharmacodynamic.

### Detection of GFP-positive cells in blood samples by flow cytometry

Ten microliters of whole blood were used to detect circulating GFP-positive cells. Briefly, blood was distributed into a 96-well round bottom plate (Costar, cat # 3795) and red blood cells (RBC) were lysed with 200 µl of red blood cell lysis buffer (Sigma, cat # R-7757). After an incubation period of 7 min. in the dark on a plate agitator, cells were centrifuged (5 min., 300 g) and the supernatant discarded by inversion of the plate. After 3 washing steps in FACS buffer (phosphate buffered saline (D-PBS), 3 % FBS and 0.02 % sodium azide), nucleated cells were resuspended in 200 µl of cold FACS buffer and processed for GFP detection using a LSRII flow cytometer (BD Biosciences, Heidelberg, Germany).

### Histology (p-STAT5, Acetyl-Histone H3, reticulin)

Sterna and spleens were collected, fixed in formalin, trimmed, embedded in paraffin and sectioned at (nominally) 4 µm using a microtome.

Bone marrow fibrosis was evaluated on the sternum using a silver impregnation kit for reticulin fibers (Bio-Optica, cat.04-04080).

The p-STAT5 PD marker of Compound A and Acetylated Histone H3 PD marker of Compound B were assessed in spleen and bone marrow. Briefly, the spleen was removed in total, weighed and placed on a dissection tray. The spleen was cut transverse in two halves and two equal pieces were taken from the middle parts using a scalpel. Spleen pieces were not allowed to exceed 3-4 mm in thickness. Spleen pieces were transferred into labeled histocassettes and immersion-fixed in neutral buffered formalin (NBF) 10 % (v/v) at room temperature (pH 6.8-7.2) (J.T. Baker, Medite, Switzerland). Fixative volume was at least tenfold in excess compared to tissue volume. Sterna were removed in total, fixed for 48 hours in 10 % NBF at room temperature then washed in PBS, decalcified in EDTA-citric acid buffer pH7.5 (Biocyc GmbH, Luckenwalde, Germany) for 3x24h at 37°C. After a last wash in PBS, the tissues were cut up and set with the surface of interest downwards in a universal histocassette followed by processing in a TPC 15Duo (Tissue Processing Center) for paraffinization. From each tissue paraffin block, several 3 µm thick sections were cut on a sliding or rotary microtome (Mikrom International AG, Switzerland), spread in a 45°C water-bath, mounted on microscope slides (Polysine, VWR International, Leuven, Belgium), and air-dried in an oven at 37°C overnight. Dry tissue section slides were taken out of the oven and placed in the slide rack of the linear stainer COT20 (Medite, Switzerland) for fully automated H&E (Haematoxylin &Eosin) staining or processed for immunohistochemistry (IHC) staining. For IHC, tissue section samples were stained with rabbit anti-phospho STAT5 antibody (clone C11C5), and rabbit anti-acetyl Histone H3 antibody (Millipore, MA, USA) coverslipped and air-dried.

For histological evaluation, BM cellularity was evaluated as hypercellularity (3+), normocellularity (2+), and hypocellularity (1+). Splenic architecture was evaluated as destroyed (1+) or preserved (0). Presence of myeloid, erythroid and adipocytes cells was evaluated as 0, 1+, 2+, 3+ based on cellularity. For p-STAT5 evaluation, digital slide image data was generated from the glass slides using the Zeiss Mirax slide scanner (Scan Software version 1.12, Zeiss AG, Germany) at a final magnification of 200x. Whole slide automated quantitative assessment of p-STAT5-positive and -negative cell nuclei were performed using Definiens eCognition software (eCognition version XD 1.5, Definiens AG, Germany). The results were expressed as percent of positive p-STAT5 nuclei out of total nuclei. Staining of acetylated histone H3 was scored as 1+, 2+, 3+ based on staining intensity and number of positive cells.

### Western blot analysis for acetylated lysine

Frozen spleen samples were homogenized in lysis buffer (RIPA buffer: 50 mM Tris-HCl pH 7.2, 120 mM NaCl, 1 mM EDTA, 6 mM EGTA pH 8.5, 1 % NP-40, 20 mM NaF supplemented with 0.1 % SDS, 2 mM sodium vanadate, 10 mM sodium pyrophosphate and one anti-proteases cocktail tablet (Roche catalogue # 11836145001) using a Polytron homogenizer (IKA LaborTechnik, Ultra-Turra T25, full speed for 1 min.), keeping samples on ice during the homogenization. Homogenates were then centrifuged at 10'000 rpm (15 min., 4°C), filtered through glass-fiber filters and frozen at -80°C. Total protein content of the homogenates was measured using the BCA protein assay kit (Novagen, catalogue # 71285-3).

100 µg of total proteins from each sample were resolved by 4-12 % Nupage gels (Invitrogen, catalogue # WG1402BX10) and transferred to PVDF membranes (Millipore Immobilon™, catalogue # IPVH20200, Billerica, Massachusetts, USA) by semi-dry blotting (BIORAD, semi dry transfer system, catalogue # 170-3940). Membranes were blocked in blocking solution (5 % BSA, 0.1 % Tween-20 in PBS) for 1 hour at room temperature followed by washing for 30 min. in PBS containing 0.1 % Tween-20 with changes every 10 min. Membranes were then incubated with primary anti-acetylated lysine antibody (rabbit polyclonal anti-acetylated lysine antibody, Cell Signaling, catalogue # 9441) diluted 1:1000 in PBS containing 0.1 %Tween-20 and 5 % milk overnight at 4°C. Next day, the membrane was washed 30 min. in PBS containing 0.5 % Tween-20 with changes every 10 min. and then incubated for 1 hour at room temperature with anti-rabbit HRP conjugated secondary antibody diluted 1:2000 in PBS containing 0.1 % Tween-20 and 1 % BSA. The membrane was washed again as above and developed with ECL+ (Amersham Biosciences, catalogue #RPN 2132) to detect acetylated-lysine in spleen extracts.

### Detection of total proteins using β-tubulin or GAPDH

For the detection of levels of total β-tubulin, the membrane was stripped in PBS containing 0.1 % Tween20 and 2 % SDS for 30 minutes at 60 °C, washed 4 to 5 times in PBS containing 0.1 % Tween20 and incubated in primary anti-β-tubulin antibody (mouse monoclonal anti-β-tubulin antibody, Sigma, cat. # T-4026) diluted 1:5000 in PBS containing 0.1 % Tween20 and 3 % BSA overnight at 4°C. Next day, the membrane was washed 30 minutes in PBS containing 0.5% Tween20 with changes every 10 minutes and then incubated for 1 hour at room temperature with anti-mouse HRP conjugated secondary antibody diluted 1:5000 in PBS containing 0.1 % Tween20 and 1 % BSA. The membrane was washed again as above and developed with ECL to detect β-tubulin protein.

GAPDH levels were detected as following: samples were reloaded (40 µg of total proteins and the membrane was incubated in primary anti-GAPDH antibody (rabbit anti-GAPDH antibody, Cell Signaling, cat. # 2118) diluted 1:5000 in PBS containing 0.1 % Tween20 and 3 % BSA overnight at 4°C. Next day, the membrane was washed 30 minutes in PBS containing 0.5% Tween20 with changes every 10 minutes and then incubated for 1 hour at room temperature with anti-rabbit HRP conjugated secondary antibody (GE Healthcare, cat # NA931) diluted 1:1000 in PBS containing 0.1 % Tween20 and 1 % BSA. The membrane was washed again as above, and developed with ECL to detect GAPDH protein in spleen extracts.

### Bioanalytics (LC/MS-MS) for quantification of Compound A and Compound B

Concentrations of Compound A and Compound B in plasma and tissues were determined simultaneously by an UPLC/MS-MS assay. Tissues were homogenized in an equal volume of HPLC-Water (Water for chromatography, Merck) using the Fast Prep®-24 system (M.P. Biomedicals, Irvine, CA, USA). Following addition of 25 µl of internal standard mixture (1 µg/ml) to analytical aliquots (25 µl) of blood or tissues homogenate, the proteins were precipitated by the addition of 200 µl acetonitrile. The supernatant were transferred in a fresh vial. After evaporation to dryness the samples were re-dissolved in 60µl acetonitrile/ water (1/1 v/v). An aliquot (5 µl) of this solution was separated on a ACQUITY UPLC BEH C18 column (Waters™ 1.7 µm particle size, 2.1 x 50 mm) with a mobile phase consisting of a mixture of 0.1 % formic acid in water (solvent A) and 0.1 % formic acid in acetonitrile (solvent B). Gradient programming was used with a flow rate of 600µl/min. After equilibration with 95% solvent A, 5 µl of sample was injected. Following a latency period of 0.25 min., the sample was eluted with a linear gradient of 5 - 100 % solvent B over a period of 0.65minutes followed by a 0.35 min. hold. The column was prepared for the next sample by re-equilibrating over 0.25 min. to the starting conditions. The column eluent was directly introduced into the ion source of the triple quadrupole mass spectrometer TQD™ (Waters Corporation, Milford, MA, USA) controlled by Masslynx™ 4.1 software. Electrospray positive ionization (ESI +) multiple reaction monitoring was used for the MS/MS detection of the analyte. Precursor to product ion transition of 307.0 --> m/z 186.0 for Compound A was used. Simultaneously precursor to product ion transition of 350.1 --> m/z 142.9 for Compound B was recorded. The limit of quantification (LOQ) for both compounds was set to 9 ng/ml and 9 ng/g for plasma and tissues, respectively (CV and overall bias less than 30 %). Regression analysis and further calculations were performed using QuanLynx™ 4.1 (Micromass) and Excel™ 2007 (Microsoft). Concentrations of unknown samples were back-calculated based on the peak area ratios of analyte/IS from a calibration curve constructed using calibration samples spiked in blank blood or tissue obtained from animals treated with vehicle.

### Maintenance Conditions

Balb/cByJIco mice (C. River, France) were held in autoclaved cages (maximum of 5 animals per cage). The light/dark cycle was as follows: 12 hours dark, 12 hours light (lights on from 6:30 AM to 6:30 PM). The animals were fed ad libitum with wetted gamma irradiated food and autoclaved water with antibiotics (BACTRIM at a final concentration of 4 mg Sulfamethoxazolime, 0.8 mg Trimethoprim), Roche Pharma AG, Reinach, Switzerland), 5 ml in 250 ml drinking water) to help recovery of animals post-irradiation and avoid infections over the first 2 weeks post-transplant.

### Test Compounds and formulation

Compound B was administered as the lactate salt by intraveneous or intraperitoneal injection. The lactic salt of Compound B was formulated in isotonic D5W (5 % Dextrose; B. Braun, Lot 395147) at a concentration of 1.5 mg/ml, 1 mg/ml and 0.5 mg/ml, respectively. The solution was stable for up to 10 days at room temperature. Treatment was given 3 times a week at a volume of 10 ml/kg. The final equivalent free-base doses were respectively 11.90, 7.94 and 3.97 mg/kg. In the figures, these doses are reported as whole values.

The mono-phosphate salt of Compound A was formulated in 0.5 % HPMC (Pharmacoat 603, Dow Chemical Plaqueline, USA) at a concentration of 7.9 mg/ml. Solution was stable at room temperature for 4 days. Treatment was given bid at a volume of 10 ml/kg. The final equivalent free-base dose was 60 mg/kg.

### The two compounds were given simultaneously.

### Statistics

Results shown in the figures and tables represent means ± SEM. Percentage change in body weights and absolute values or transformed values (log 10, or other as specified) for spleen weights, reticulocyte, WBC counts, Hct and histological data were analyzed by unpaired t-test or Rank-Sum test to compare a single treatment group to vehicle group or one way ANOVA followed by Dunnett's test to compare treatment groups to vehicle group. Multiple comparisons were done using the Tukey's test. All comparisons were done on sacrifice day. The significance level was set to p < 0.05. Calculations were performed using GraphPad Prism for Windows (GraphPad Software Inc.).

### Results of Example B:

### In vivo efficacy and tolerability of Compound A and Compound B in combination

To test the hypothesis that efficacy of Compound B could be improved by combination with Compound A, Balb/c mice transplanted with JAK2V617F transduced bone marrow cells were treated with Compound A in combination with the JAK1/2 inhibitor Compound A. A dose giving an intermediate efficacy was chosen for Compound A in order to assess the impact of different doses of Compound B in combination with the JAK inhibitor Compound A.

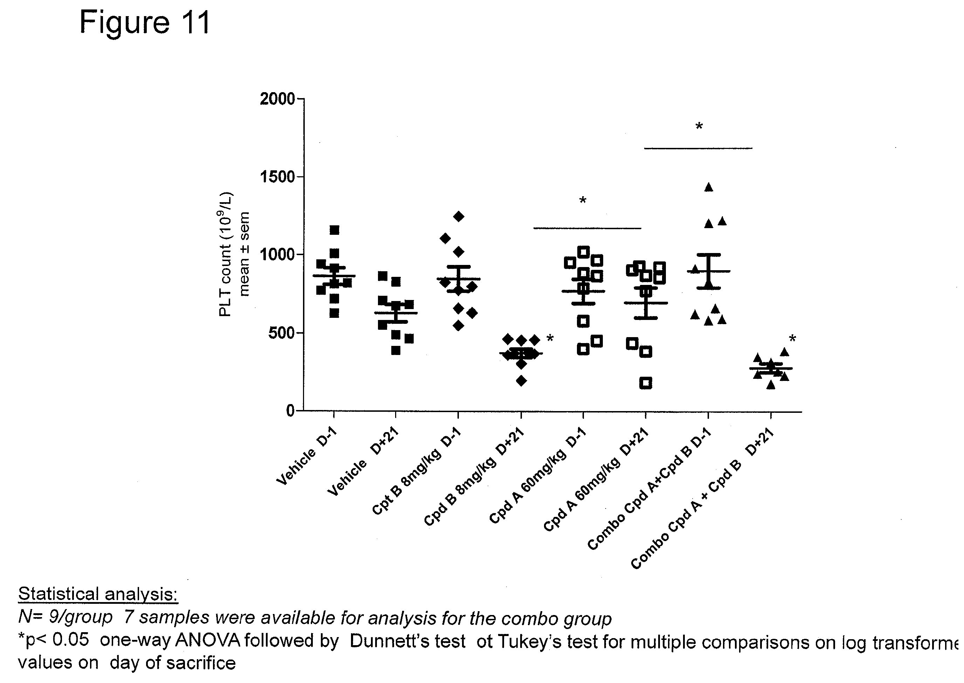

Mice were randomized on day 27 post BMT based on Hct values (67 % in average in this experiment, N=9/group) and treated with Compound B (8 mg/kg, 3x/week MWF, i.p.) and Compound A (60 mg/kg, q12h, p.o.) as single agents or combined. Compound B alone showed some body weight loss (-5 % on average). This body weight loss was significantly higher when Compound B was combined with Compound A (-10% on average, Figure 7 and Table 4). Compound B given alone reduced spleen weight but not to normal. Compound A showed trend for a reduction in spleen weight but with high variability (range 67 to 1153 mg). The combination improved efficacy in terms of spleen weight/volume, which normalized or was even below normal historic range (in 6/9 animals) after 3 weeks of treatment (Figure 8 and Table 4). The combination showed a trend for stronger effect on reticulocyte count (with very low values observed for some animals < 0.1x10¹²/L) but this effect was not significantly different from single agent treated groups. WBC count was reduced when Compound B was given alone and in combination with Compound A (despite the absence of leukocytosis in this experiment except for 1 animal in the vehicle and 1 animal in the Compound A group). PLT count was impacted by Compound B treatment alone and in combination with Compound A (Figure 11). A trend for a reduction in the allele burden surrogate readout (GFP-positive circulating cells) was observed in this study for Compound B and the combination groups (Table 4).

A decrease in bone marrow hypercellularity was observed for all drug treatment groups of treatment (Combo > Compoud A = Compound B). Treatment improved splenic architecture with the strongest effect observed in the combination group (Combo> Compound A > Compound B) Despite high variability, the Compound A and the combination groups showed a tendency to reduce the fibrosis score, as assess by reticulin staining on sternum sections.

PD marker assessment by IHC showed a clear reduction in the p-STAT5 marker upon treatment with Compound A as a single agent and in combination with Compound B. A clear increase was seen for acetylated histone H3, as surrogate readout for deacetylase inhibition, upon treatment with Compound B alone or in combination.

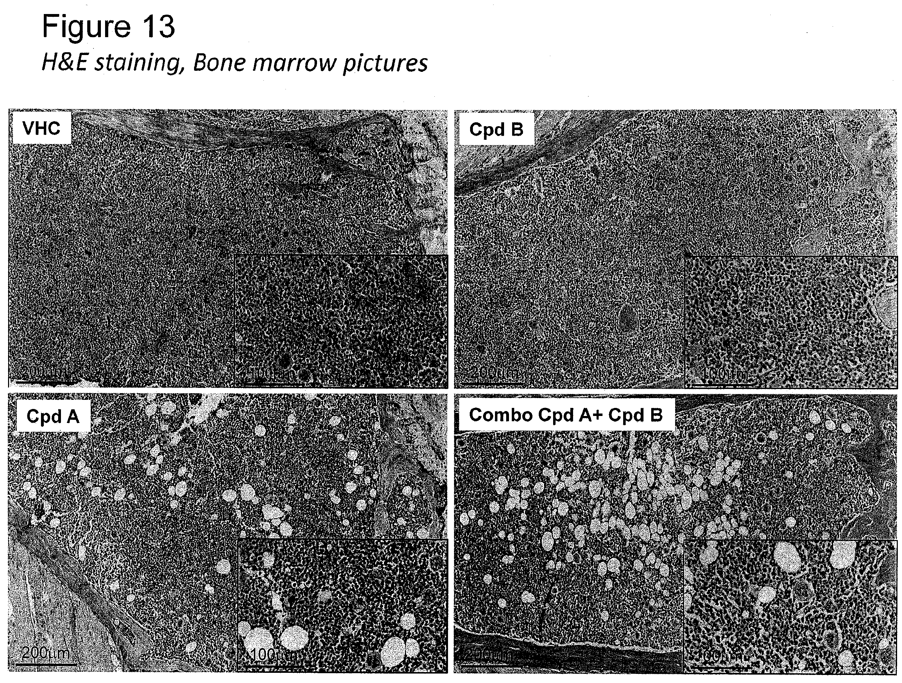

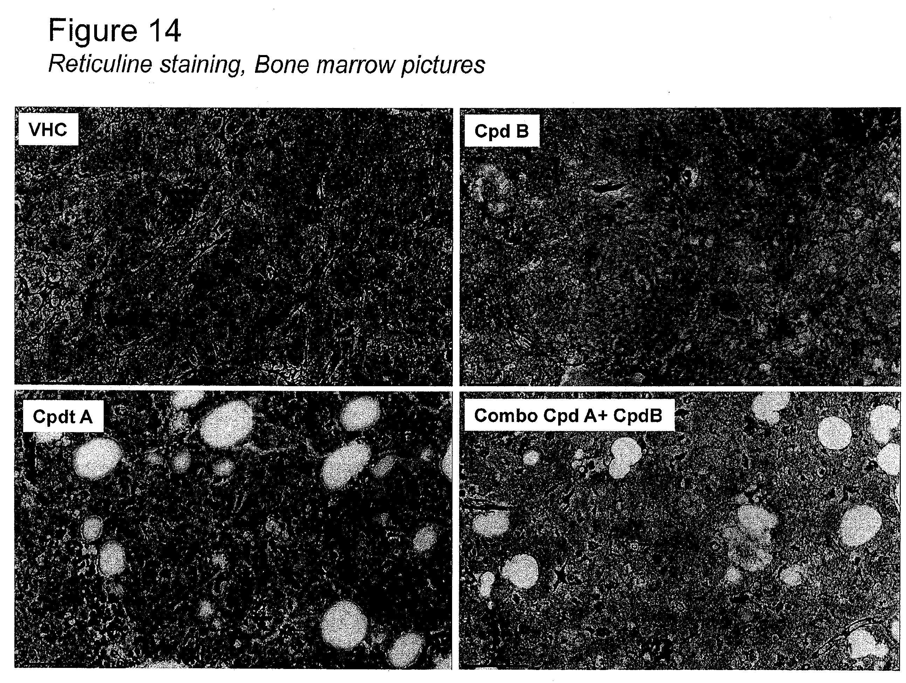

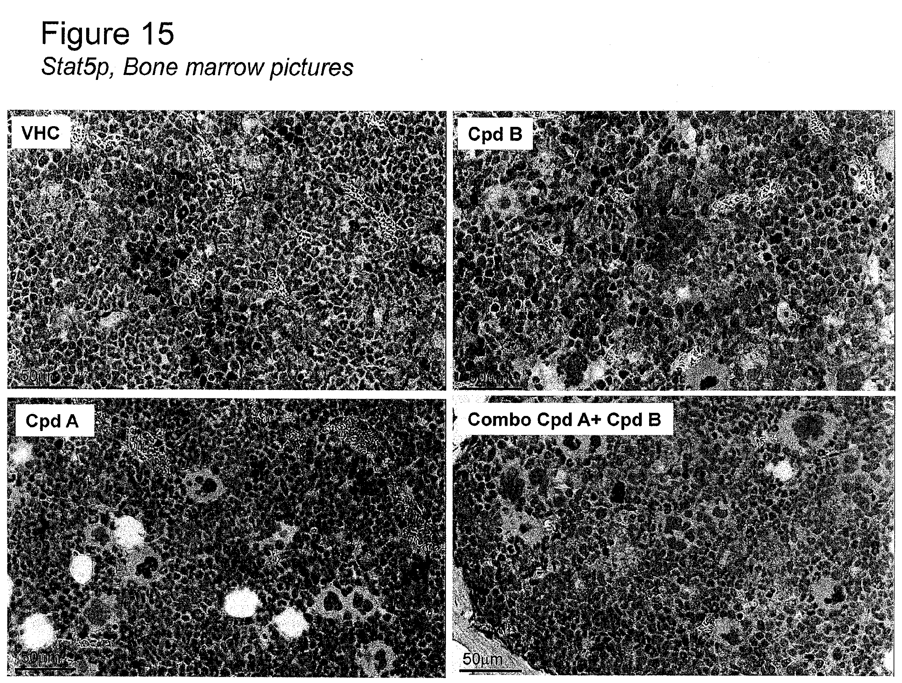

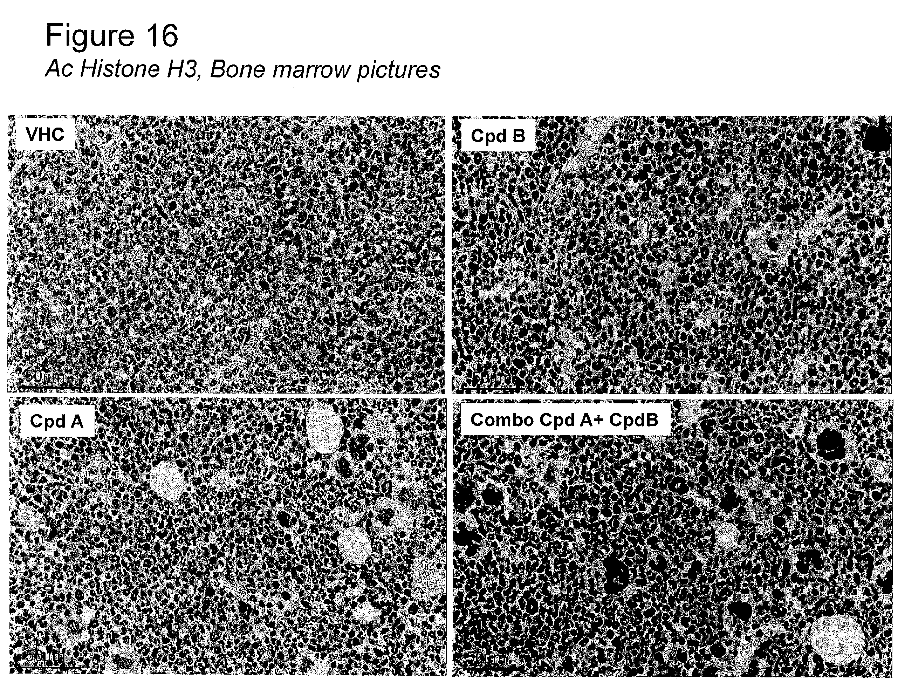

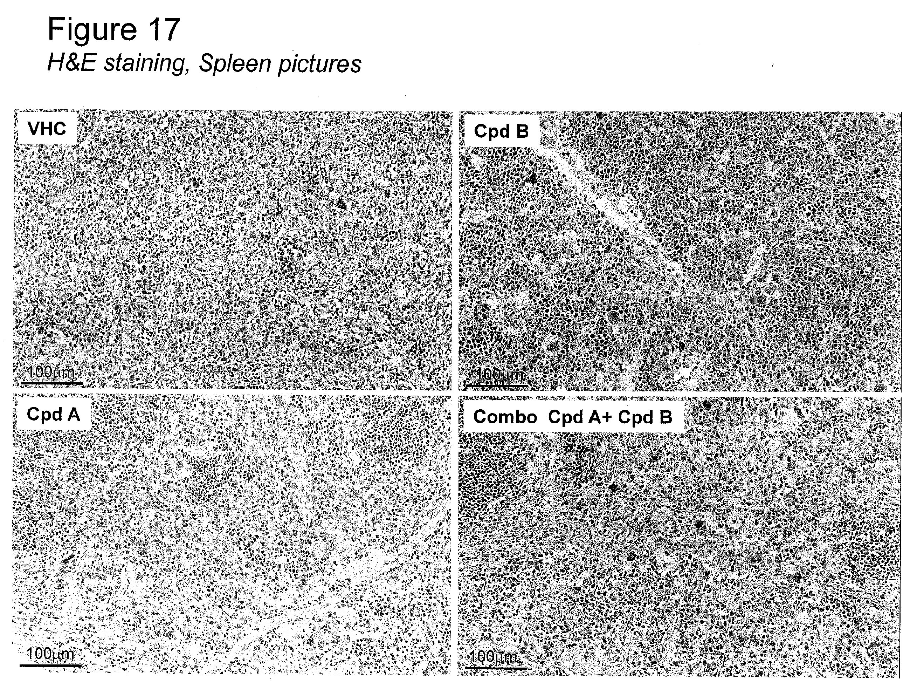

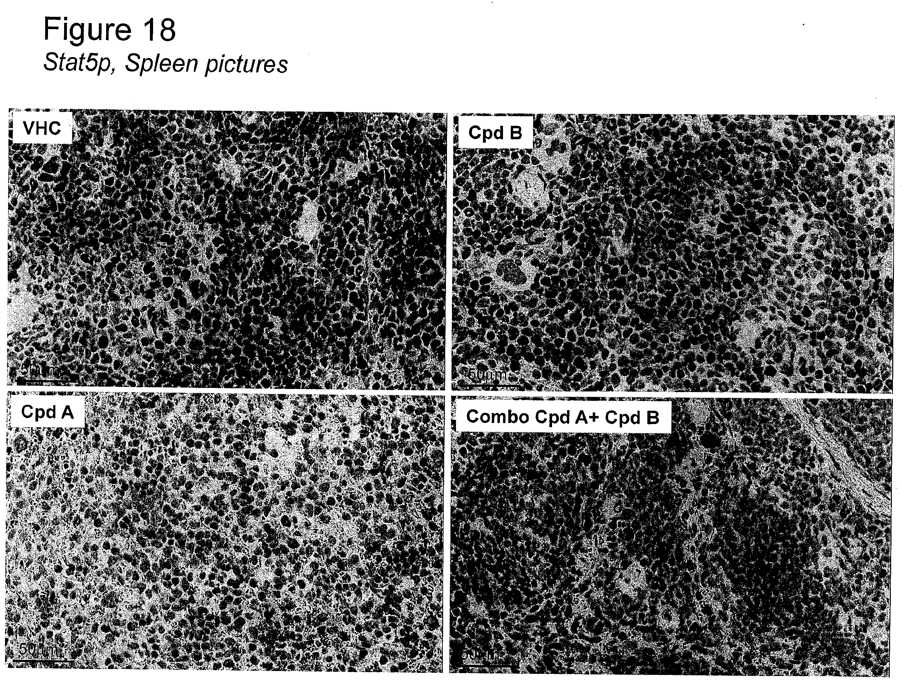

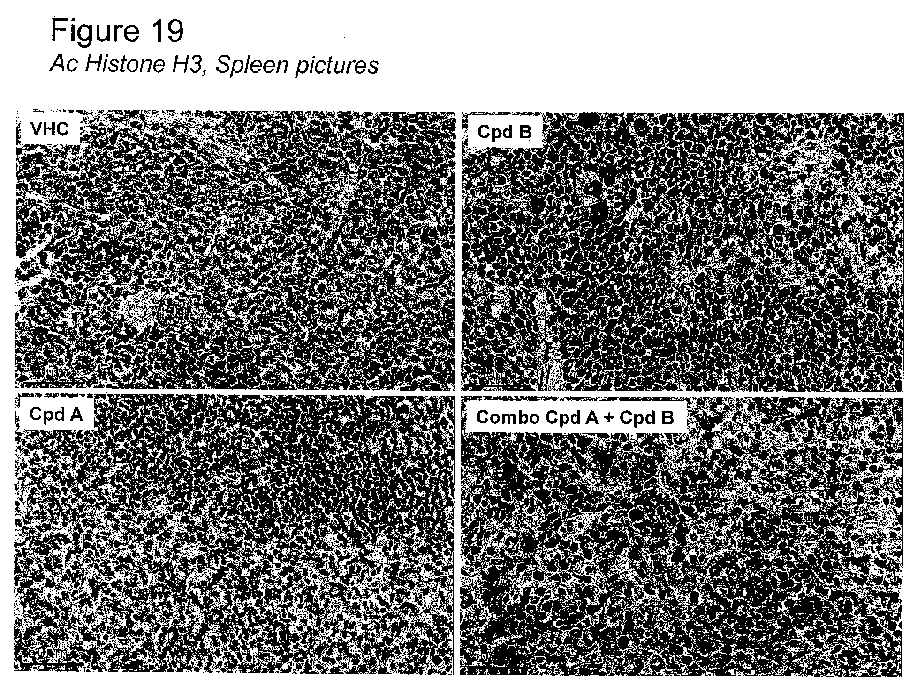

Figures 13-19 depict bone marrow and/or spleen images under different staining methods.

There was no major impact of the combination regimen on the exposure of the compounds in tissues (Table 5).

This example demonstrates that the combination of Compounds A and B improved efficacy in terms of spleen weight/volume.

**Table 4. Results on sacrifice day**

| **Treatment** | **BW change (%)** | **Spleen weight** | **WBC** | **PLT** | **Retic counts** | **Hct** | **GFP-positive circulating cells (%)** |
|---|---|---|---|---|---|---|---|
| | | **(mg)** | **(x 10⁹/L)** | **(x 10⁹/L)** | **(x 10¹²/L)** | **(%)** | |
| | **(mean ± SEM)** | **(mean± SEM)** | **(mean ± SEM)** | **(mean ± SEM)** | **(mean ± SEM)** | **(mean ± SEM)** | |
| **Vehicle D5W i.p. and HPMC 0.5 % p.o.** | 2.01 ± 1.18 | 519.9 ± 121.8 | 53.0 ± 41.9 (11.15 ± 0.76) | 628.6 ± 54.2 | 0.57 ± 0.11 | 66.5 ± 4.2 | 33.4 ± 9.1 |
| **10 mg/kg** | | | | | | | |
| **Compound B (3 times a week, ip, 8mg/kg)** | -4.57 ± 1.14* | 222.0 ± 92.3* | 3.88 ± 0.94* | 374.4 ± 28.6* | 0.24 ± 0.10* | 52.2 ± 3.1* | 20.4 ± 6.6 |
| **Compound A (q12h, po, 60mg/kg)** | 0.45 ± 0.94 | 346.1 ± 127.9 | 22.2 ± 14.7 (7.46 ± 0.82) | 695.9 ± 93.6 | 0.42 ± 0.09 | 59.4 ± 3.9 | 34.0 ± 9.6 |
| **Combo Compound B (8 mg/kg) + Compound A (60 mg/kg)** | -9.52 ± 0.55* | 71.9 ± 8.6 *^{†#} | 2.41 ± 0.48*^{#} (N=7) | 280.1 ±28.0*^{#} (N=7) | 0.11 ±0.04* (N=7) | 51.5 ±3.6* (N=7) | 16.8 ± 4.3 (N=7) |

| | | | | | | | |
|---|---|---|---|---|---|---|---|
| Balb/c female mice transplanted with JAK2^{V617F} bone marrow transduced cells received either vehicle, Compound B at a dose of 8 mg/kg i.p. (free-base equivalent) on a M/W/F schedule, Compound A at dose of 60 mg/kg (free-base equivalent) q12h or the combination of both agents for 21 consecutive days. Changes in body weight and spleen weight at sacrifice are depicted as mean ± SEM. N = 7-9/group.*p < 0.05 vs. vehicle, # p < 0.05 vs. Compound A, ^{†}p < 0.05 vs. Compound B on sacrifice day (one way ANOVA followed by Dunnett's test or Tukey's test.The reciprocal form of the spleen weight values and log₁₀ transformed values for WBC and PLT counts were used for statistical analysis). 1 outlier in the vehicle (388.3 x10⁹/L) and Compound A (139.9x10⁹/L) groups for WBCs counts was detected new mean ± SEM values without outliers given between brackets. | | | | | | | |

**Table 5. Levels post therapy**

| | **Blood** | | **Spleen** | | **Liver** | | **BM** | |
|---|---|---|---|---|---|---|---|---|
| | **µmol/L** | | **nmol/g** | | **nmol/g** | | **nmol/g** | |
| **Treatment** | Comp'd A | Comp'd B | Comp'd A | Comp'd B | Comp'd A | Comp'd B | Comp'd A | Comp'd B |
| **Comp'd B** | - | 0.035 ± 0.010 | - | 10.045 ± 1.674 | - | 0.264 ± 0.045 | - | 2.704 ± 0.345 |
| **8 mg/kg** | | | | | | | | |
| **2 hr** | | | | | | | | |
| **Comp'd A** | 8.176 ± 1.584 | - | 2.566 ± 0.346 | - | 6.537 ± 1.071 | - | 2.223 ± 0.393 | - |
| **60 mg/kg** | | | | | | | | |
| **2 hr** | | | | | | | | |
| **Combo Compound A and B** | 4.114 ± 0.979 | 0.006 ± 0.004 | 1.575 ± 0.312 | 15.317 ± 1387 | 3.568 ± 0.730 | 0.33 ± 0.039 | 1.469 ± 0.318 | 4.398 ± 0.413 |
| **2 hr** | | | | | | | | |

Animals in the respective dosing groups were sacrificed 2 hours after the final dose of Compound B and/or Compound A and blood, spleen, bone marrow and liver were sampled for PK analysis. Doses are reported here as free-base equivalent. The table shows mean Compound B and Compound A levels in blood [µmol/l] and tissues [nmol/g] ± SEM. N=9/group.

### EXAMPLE C

### Clinical Trial

A Phase 1b, open-label, multi-center, single arm, dose finding study to assess safety and pharmacokinetics of the oral combination of panobinostat and ruxolitinib in patients with primary myelofibrosis (PMF), post-polycythemia vera-myelofibrosis (PP-MF) or post-essential thrombocythemia-myelofibrosis (PET-MF) is in progress

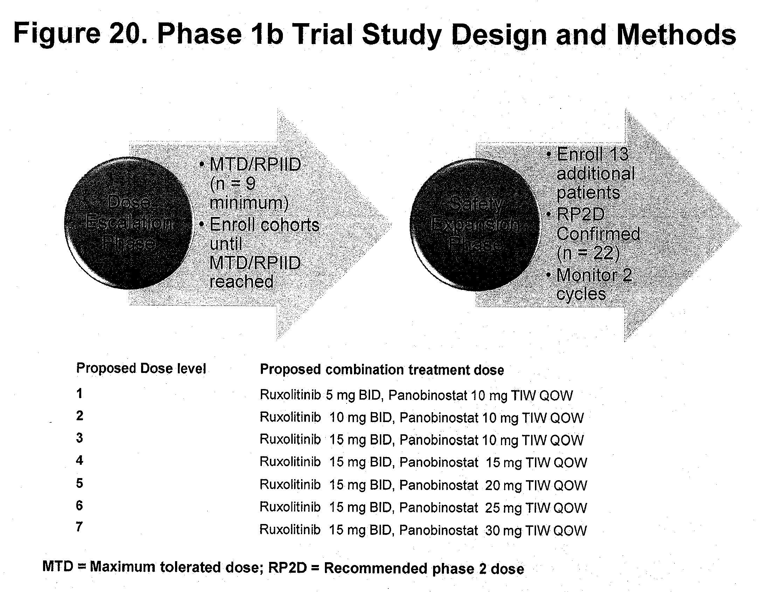

Figure 20 illustrates the study design and methods.

This trial aims to establish one or more of the following:
(1) To establish the MTD and/or RPIID of the combination of ruxolitinib and panobinostat in patients with MF;
(2) To evaluate the safety of the oral co-administration of ruxolitinib and panobinostat to patients with MF;
(3) To characterize the pharmacokinetics of ruxolitinib at varying doses, - as a single agent, and when given in combination with panobinostat to patients with MF; and/or
(4) To characterize the pharmacokinetics of panobinostat, at varying doses in combination with ruxolitinib in patients with MF.

### Inclusion Criteria:

A. Patients had a documented diagnosis of PMF, PPV-MF, or PET-MF, irrespective of their JAK2 V617F mutation status
B. Guided by the World Health Organization (WHO) criteria for PMF, the study includes patients designated as Intermediate-1, -2, or high risk by International Prognostic Scoring System (IPSS) criteria and who have palpable splenomegaly ≥ 5cm below the costal margin
C. Patients must have had at least 1 of the following risk factors
   a. Presence of constitutional symptoms (weigh loss > 10% of the baseline value in the year preceding cycle 1, day 1 [C1D1], unexplained fever, or excessive night sweats persisting for more than 1 month)
   b. Marked anemia (hemoglobin < 10 g/dL0 demonstrated at the screening visit
   c. Leukocytosis (history of white blood cell count > 25 x 10⁹/L)
   d. Circulating blasts ≥ 1%
D. Dose escalation is guided by a Bayesian logistic regression model with overdose control and will depend on dose-limiting toxicities (DLTs) in the first cycle as well as other safety findings
E. Each dosing cohort consisted of ≥ 3 evaluable patients
F. Data for ≥ 9 patients at any given dose level will be required to determine the RP2D and/or MTD
G. Serial blood samples collected following a single dose of ruxolitinib alone on day 1 and in combination with panobinostat on days 2 and 6 are evaluated for plasma concentrations by liquid chromatography-tandem mass spectrometry
H. Pharmacokinetic parameters were derived using noncompartmental analysis

### Results:

7 cohorts are proposed in the study. The study is still on going, only data from Cohorts 1-3 are available to date.

Table 6 shows the Patient number and disease subtype for Cohorts 1-3.

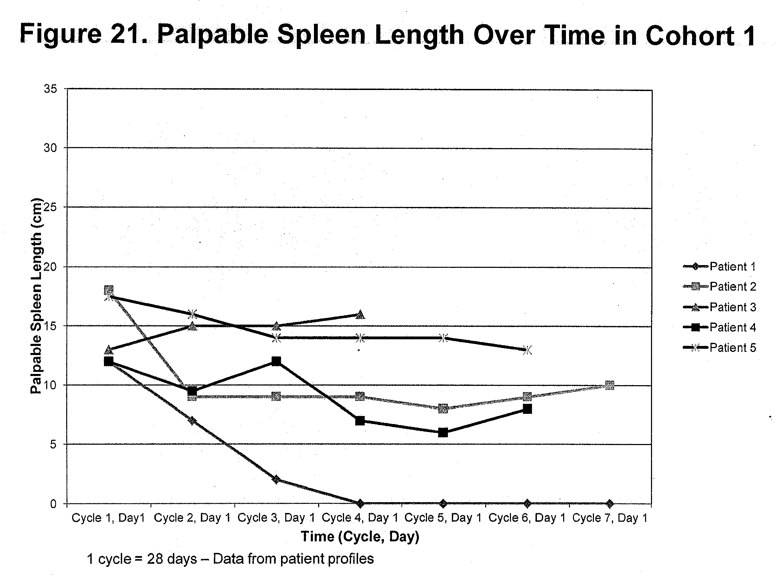

Figure 21 illustrates the palpable spleen length over time in Cohort 1.

Table 7 shows the best palpable spleen length response and symptom response in Cohort 1.

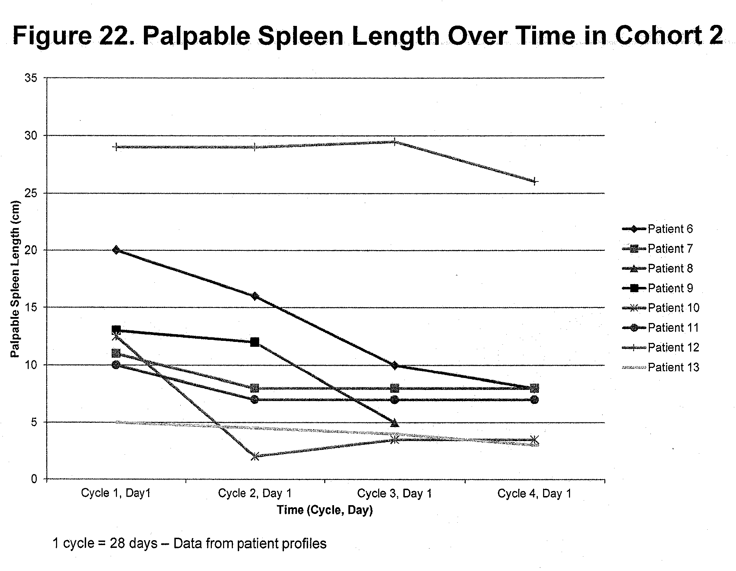

Figure 22 illustrates the palpable spleen length over time in Cohort 2.

Table 8 shows the best palpable spleen length response and symptom response in Cohort 2.

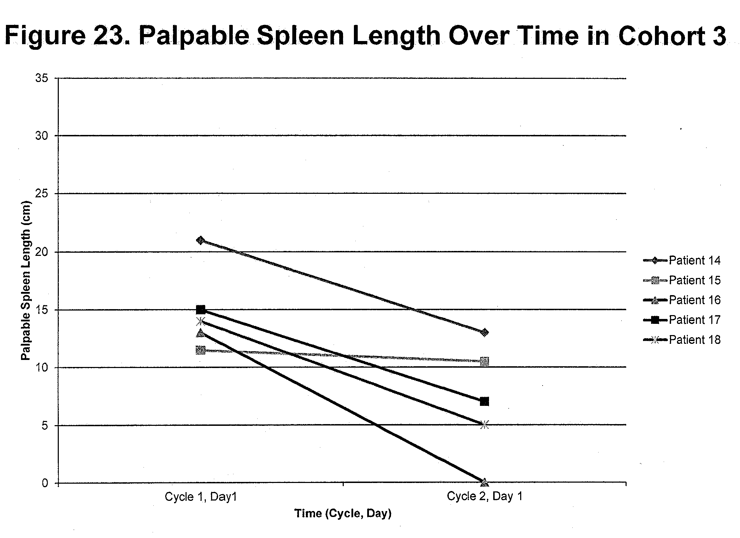

Figure 23 illustrates the palpable spleen length over time in Cohort 3.

Table 9 shows the best palpable spleen length response and symptom response in Cohort 3.

Table 10 reports Grade 3 / 4 adverse events within Cohorts 1-3 with suspected relationship to study treatments. There have been no DLTs or SAEs observed in cohort 1 or cohort 3. In cohort 2, there was 1 DLT of grade 4 thrombocytopenia reported. There was also 1 SAE of grade 3 nausea and grade 3 diarrhea. No clinically significant EKG abnormalities have been observed to date.

**Table 6. Ruxolitinib and Panobinostat Dosages in Cohorts 1, 2, and 3**

| Dose escalation is guided by a Bayesian logistic regression model with overdose control and will depend on dose-limiting toxicities (DLTs) in each cycle as well as other safety findings Each dosing cohort consisted of ≥ 3 evaluable patients Data for ≥ 9 patients at any given dose level will be required to determine the RP2D and/or MTD | | | | | | |
|---|---|---|---|---|---|---|
| Serial blood samples collected following a single dose of ruxolitinib alone on day 1 and in combination with panobinostat on days 2 and 6 are evaluated for plasma concentrations by LC-MS/MS | | | | | | |
| PK parameters were derived using noncompartmental analysis | | | | | | |
| Cohort | # of Patients by Disease Subtype | | | # of Patients Total | Therapeutic Dosage | |
| | PMF | PET-MF | PPV-MF | | Ruxolitinib | Panobinostat |
| 1 | 2 | 3 | 0 | 5 | 5 mg BID | 10 mg TIW/QOW |
| 2 | 3 | 2 | 3 | 8 | 10 mg BID | 10 mg TIW/QOW |
| 3 | 3 | 2 | 0 | 5 | 15 mg BID | 10 mg TIW/QOW |

**Table 7. Best Palpable Spleen Length Response and Symptom Response in Cohort 1**

| | **Palpable Spleen Length Best Response** | **Change in Constitutional Symptoms** | | |
|---|---|---|---|---|
| | | **10% Weight Loss From BL** | **Night Sweats Persisting for > 1 Month** | **Unexplained Fever > 37.5 °C Persisting for > 1 Month** |
| **Cohort 1 (n = 5) Data from patient profiles** | | | | |
| **Patient 1** | **100% reduction** | **--** | **Resolved** | **--** |
| **Patient 2** | **56% reduction** | **No change** | **Resolved** | **--** |
| **Patient 3a** | **23% increase** | **--** | **Resolved** | **Resolved** |
| **Patient 4** | **50% reduction** | **--** | **Resolved** | **--** |
| **Patient 5** | **26% reduction** | **No change** | **Resolved** | **--** |

| | | | | |
|---|---|---|---|---|
| ^{a} Patient experienced rising blast counts and was taken off study to undergo stem cell transplantation | | | | |

| **Table 8. Best Palpable Spleen Length Response and Symptom Response in Cohort 2** | | | | |
|---|---|---|---|---|
| | | | | |
| | **Palpable Spleen Length Best Response** | **Change in Constitutional Symptoms** | | |
| | | **10% Weight Loss From BL** | **Night Sweats Persisting for > 1 Month** | **Unexplained Fever >37.5°C Persisting for > 1 Month** |
| **Cohort 2 (n = 8) Data from patient profiles** | | | | |
| **Patient 6** | **60% reduction** | **No change** | **--** | -- |
| **Patient 7** | **27% reduction** | -- | **Resolved** | -- |
| **Patient 8** | **62% reduction** | -- | **Resolved** | -- |
| **Patient 9a** | **8% reduction** | -- | **Resolved** | -- |
| **Patient 10** | **84% reduction** | **No change** | **Resolved** | -- |
| **Patient 11** | **30% reduction** | -- | **Resolved** | -- |
| **Patient 12** | **10% reduction** | **No change** | **Resolved** | -- |
| **Patient 13** | **40% reduction** | **No change** | **Resolved** | -- |
| ^{a} Patient experienced grade 4 thrombocytopenia and was taken off study due to DLT criteria | | | | |

**Table 9. Best Palpable Spleen Length Response and Symptom Response in Cohort 3 (dashes indicate that this symptom is not present at baseline)**

| | **Palpable Spleen Length Best Response** | **Change in Constitutional Symptoms** | | |
|---|---|---|---|---|
| | | **10% Weight Loss From BL** | **Night Sweats Persisting for > 1 Month** | **Unexplained Fever >37.5°C Persisting for > 1 Month** |
| **Cohort 3 (n = 5) Data from patient profiles** | | | | |
| **Patient 14** | **38% reduction** | **--** | **Resolved** | -- |
| **Patient 15** | **9% reduction** | **--** | **Resolved** | -- |
| **Patient 16a** | **100% reduction** | **No change** | **--** | -- |
| **Patient 17** | **53% reduction** | **No change** | **--** | -- |
| **Patient 18** | **64% reduction** | **Not reported at study entry** | | |

**Table 10. Grade 3/4 Adverse Events Within Cohorts 1, 2, and 3 With Suspected Relationship to Study Treatment^{a,b}**

| | **Cohort 1** | **Cohort 2** | **Cohort 3** | |
|---|---|---|---|---|
| | **PAN 10mg** | **PAN 10mg** | **PAN 10mg** | **All dose** |
| | **RUX 5mg** | **RUX 10mg** | **RUX 15mg** | **groups** |
| | **N=5** | **N=8** | **N=5** | **N = 18** |
| **Total** | 1 | 3 | 2 | 6 |
| **Total blood and lymphatic system disorders, n** | 1 | 2 | 2 | 5 |
| **Thrombocytopenia, n** | 0 | 1 | 1 | 2 |
| **Anemia, n** | 1 | 1 | 2 | 4 |
| **Total gastrointestinal disorders, n** | 0 | 1 | 0 | 1 |
| **Diarrhea, n** | 0 | 1 | 0 | 1 |
| **Nausea, n** | 0 | 1 | 0 | 1 |

| | | | | |
|---|---|---|---|---|
| PAN, panobinostat; RUX, ruxolitinib ^{a}Patients with multiple AEs within a primary system organ class were counted only once in the total row. ^{b}Patients with multiple occurrences of an AE under one treatment are counted only once in the AE category for that treatment. | | | | |

There have been no DLTs or SAEs observed in cohort 1 or cohort 3
In cohort 2, there was 1 DLT of grade 4 thrombocytopenia reported
There was also 1 SAE of grade 3 nausea and grade 3 diarrhea
No clinically significant EKG abnormalities have been observed to date

The clinical study to date shows that the combination of ruxolitinib and panobinostat appears to be well tolerated with promising activity. Low rates of grade 3/4 anemia and thrombocytopenia have been observed at the ruxolitinib and panobinostat doses explored thus far. Early data suggest no potential drug interaction between ruxolitinib and panobinostat. Additional cohorts will establish the optimal dosing strategy for this promising combination in the treatment of MF patients.

### List of abbreviations:

| **Abbreviation** | **Description** |
|---|---|
| Bid | Bis in diem |
| BMT | Bone marrow transplant |
| TIW | Three time a week |
| QOW | Every other week |
| CBC | Complete blood count |
| FACS | Fluorescence-activated cell sorter |
| FBS | Foetal bovine serum |
| FCS | Foetal calf serum |
| 5-FU | 5-Fluorouracil |
| GFP | Green fluorescent protein |
| Gy | Gray |
| Hct | Hematocrit |
| H&E | Hematoxylin/Eosin staining |
| Hr | hour |
| IHC | Immunohistochemistry |
| i.p. | Intraperiteoneal injection |
| i.v. | Intravenous injection |
| IS | internal standard |
| mlL3 | Murine Interleukin 3 |
| mlL6 | Murine Interleukin 6 |
| min | minute |
| JAK1/2 | Janus kinase 1/2 |
| MPN | Myeloproliferative neoplasm |
| MWF | Monday/Wednesday/Friday schedule |
| PD | Pharmacodynamic |
| PK | Pharmacokinetic |
| PLT | Platelets |
| PV | Polycythemia vera |
| p.o. | Per os |
| RBC | Red blood cells |
| SCF | Stem Cell Factor |
| STAT5 | Signal transducer and activator of transcription 5 |
| VHC | Vehicle |
| WBC | White blood cells |
| Wt | Wild type |

## Claims

1. A composition comprising Compound A ((R)-3-(4-(7H-pyrrolo[2,3-d]pyrimidin-4-yl)-1H-pyrazol-1-yl)-3-cyclopentylpropanenitrile) of Formula (A): 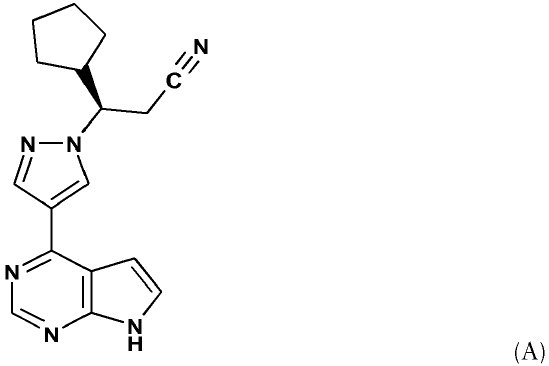 or a pharmaceutically acceptable salt thereof; and
Compound B (*N*-hydroxy-3-[4-[[[2-(2-methyl-1*H*-indol-3-yl)-ethyl]-amino]methyl]phenyl]-2*E*-2-propenamide) of Formula (B): 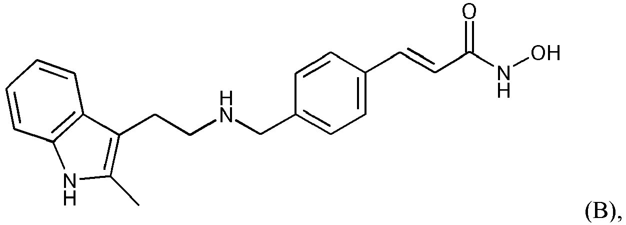 or a pharmaceutically acceptable salt thereof, for use in the treatment of a cancer, wherein the cancer is myeloproliferative neoplasm or multiple myeloma.

2. The composition for use according to claim 1, further comprising pharmaceutically acceptable carrier(s).

3. The composition for use according to claim 1 wherein the composition is in a single formulation or unit dosage form.

4. The composition for use in the treatment of cancer according to claim 1, wherein the cancer is multiple myeloma.

5. The composition for use in the treatment of cancer according to claim 1, wherein the cancer is a myeloproliferative neoplasm.

6. The composition for use in the treatment of cancer according to claim 5, wherein the myeloproliferative neoplasm is selected from the group consisting of chronic myeloid leukemia (CML), polycythemia vera (PV), essential thrombocythemia (ET), primary or idiopathic myelofibrosis (PMF), chronic neutrophilic leukemia, chronic eosinophilic leukemia, chronic myelomonocytic leukemia, juvenile myelomonocytic leukemia, hypereosinophilic syndrome, systemic mastocytosis, and atypical chronic myelogenous leukemia.

7. The composition for use in the treatment of cancer according to any of claims 4-6, wherein the composition is to be administered to a human.

8. The composition for use in the treatment of cancer according to any of claims 4-6, wherein Compound A and Compound B are to be co-administered.

9. The composition for use in the treatment of cancer according to any of claims 4-6, wherein Compound A and Compound B are in a single formulation or unit dosage form.

10. The composition for use in the treatment of cancer according to any of claims 4-6, wherein the Compound A and Compound B are to be administed at substantially the same time.

11. The composition for use in the treatment of cancer according to any of claims 4-6, wherein the Compound A and Compound B are to be administered at different times.

12. The composition for use in the treatment of cancer according to any of claims 8-10, wherein Compound A and Compound B are in separate formulations or unit dosage forms.

## Patentansprüche

1. Zusammensetzung, umfassend Verbindung A ((R)-3-(4-(7H-Pyrrolo[2,3-d]pyrimidin-4-yl)-1H-pyrazol-l-yl)-3-cyclopentylpropannitril) der Formel (A): 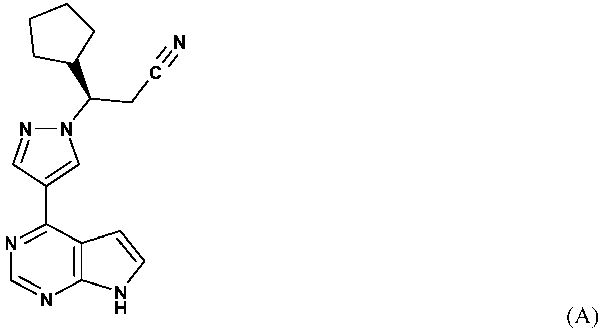 oder ein pharmazeutisch unbedenkliches Salz davon und
Verbindung B (*N*-Hydroxy-3-[4-[[[2-(2-methyl-1H-indol-3-yl)ethyl]amino]methyl]phenyl]-2*E*-2-propenamid) der Formel (B): 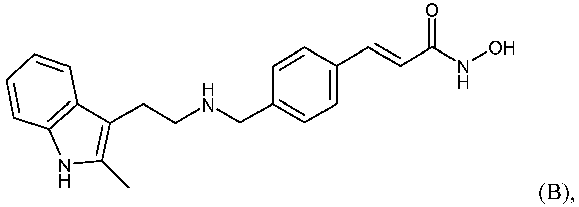 oder ein pharmazeutisch unbedenkliches Salz davon, zur Verwendung in der Behandlung eines Krebs, wobei es sich bei dem Krebs um myeloproliferatives Neoplasma oder multiples Myelom handelt.

2. Zusammensetzung zur Verwendung nach Anspruch 1, die weiterhin (einen) pharmazeutisch unbedenkliche(n) Träger umfasst.

3. Zusammensetzung zur Verwendung nach Anspruch 1, wobei die Zusammensetzung in Einzelformulierungs- oder Dosierungseinheitsform vorliegt.

4. Zusammensetzung zur Verwendung in der Behandlung von Krebs nach Anspruch 1, wobei es sich bei dem Krebs um multiples Myelom handelt.

5. Zusammensetzung zur Verwendung in der Behandlung von Krebs nach Anspruch 1, wobei es sich bei dem Krebs um ein myeloproliferatives Neoplasma handelt.

6. Zusammensetzung zur Verwendung in der Behandlung von Krebs nach Anspruch 5, wobei das myeloproliferative Neoplasma aus der Gruppe bestehend aus chronischer myeloischer Leukämie (CML), Polycythemia vera (PV), Essentieller Thrombozythämie (ET), primärer oder idiopathischer Myelofibrose (PMF), chronischer Neutrophilenleukämie, chronischer Eosinophilenleukämie, chronischer Myelomonozytenleukämie, juveniler Myelomonozytenleukämie, Hypereosinophilie-Syndrom, systemischer Mastozytose und atypischer chronischer myeloischer Leukämie ausgewählt ist.

7. Zusammensetzung zur Verwendung in der Behandlung von Krebs nach einem der Ansprüche 4-6, wobei die Zusammensetzung einem Menschen verabreicht werden soll.

8. Zusammensetzung zur Verwendung in der Behandlung von Krebs nach einem der Ansprüche 4-6, wobei Verbindung A und Verbindung B gemeinsam verabreicht werden sollen.

9. Zusammensetzung zur Verwendung in der Behandlung von Krebs nach einem der Ansprüche 4-6, wobei Verbindung A und Verbindung B in Einzelformulierungs- oder Dosierungseinheitsform vorliegen.

10. Zusammensetzung zur Verwendung in der Behandlung von Krebs nach einem der Ansprüche 4-6, wobei Verbindung A und Verbindung B im Wesentlichen zum selben Zeitpunkt verabreicht werden sollen.

11. Zusammensetzung zur Verwendung in der Behandlung von Krebs nach einem der Ansprüche 4-6, wobei Verbindung A und Verbindung B zu unterschiedlichen Zeitpunkten verabreicht werden sollen.

12. Zusammensetzung zur Verwendung in der Behandlung von Krebs nach einem der Ansprüche 8-10, wobei Verbindung A und Verbindung B in getrennten Formulierungen oder Dosierungseinheitsformen vorliegen.

## Revendications

1. Composition comprenant le Composé A ((R)-3-(4-(7H-pyrrolo[2,3-d]pyrimidin-4-yl)-1H-pyrazol-1-yl)-3-cyclopentylpropanenitrile) de Formule (A) : 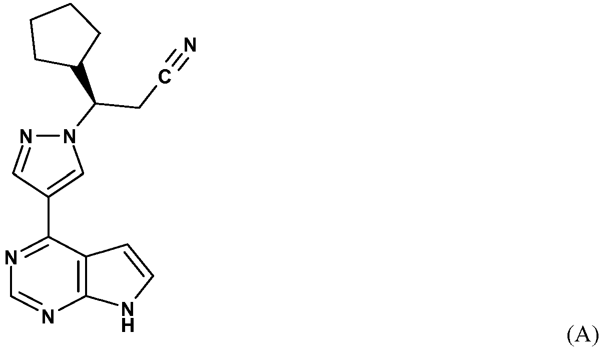 ou l'un des sels pharmaceutiquement acceptables de celui-ci ; et
le Composé B (*N*-hydroxy-3-[4-[[[2-(2-méthyl-1*H*-indol-3-yl)-éthyl]-amino]méthyl]phényl]-2*E*-2-propénamide) de Formule (B) : 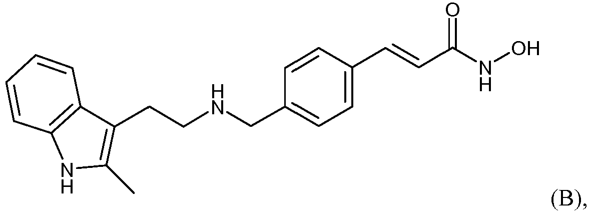 ou l'un des sels pharmaceutiquement acceptables de celui-ci, pour utilisation dans le traitement d'un cancer, où le cancer est une néoplasie myéloproliférative ou un myélome multiple.

2. Composition pour utilisation selon la revendication 1, comprenant en outre un ou plusieurs vecteurs pharmaceutiquement acceptables.

3. Composition pour utilisation selon la revendication 1, où la composition se présente sous la forme d'une formule ou d'une forme galénique unitaire unique.

4. Composition pour utilisation dans le traitement du cancer selon la revendication 1, où le cancer est un myélome multiple.

5. Composition pour utilisation dans le traitement du cancer selon la revendication 1, où le cancer est une néoplasie myéloproliférative.

6. Composition pour utilisation dans le traitement du cancer selon la revendication 5, où la néoplasie myéloproliférative est choisie dans le groupe constitué par les suivantes : leucémie myéloïde chronique (LMC), polyglobulie essentielle (PE), thrombocytopénie essentielle (TE), myélofibrose primitive ou idiopathique (MPF), leucémie chronique à neutrophiles, leucémie chronique à éosinophiles, leucémie myélomonocytaire chronique, leucémie myélomonocytaire juvénile, syndrome hyperéosinophile, mastocytose systémique, et leucémie myéloïde chronique atypique.

7. Composition pour utilisation dans le traitement du cancer selon l'une quelconque des revendications 4 à 6, où la composition doit être administrée à un humain.

8. Composition pour utilisation dans le traitement du cancer selon l'une quelconque des revendications 4 à 6, où le Composé A et le Composé B doivent être co-administrés.

9. Composition pour utilisation dans le traitement du cancer selon l'une quelconque des revendications 4 à 6, où le Composé A et le Composé B se présentent sous la forme d'une formule ou d'une forme galénique unitaire unique.

10. Composition pour utilisation dans le traitement du cancer selon l'une quelconque des revendications 4 à 6, où le Composé A et le Composé B doivent être administrés substantiellement simultanément.

11. Composition pour utilisation dans le traitement du cancer selon l'une quelconque des revendications 4 à 6, où le Composé A et le Composé B doivent être administrés à des instants différents.

12. Composition pour utilisation dans le traitement du cancer selon l'une quelconque des revendications 8 à 10, où le Composé A et le Composé B se présentent sous la forme de formules ou de formes galéniques unitaires distinctes.
